(19) Europäisches Patentamt
European Patent Office
Office européen des brevets

(11) EP 3 483 242 A1

(12) EUROPEAN PATENT APPLICATION

(43) Date of publication:
**15.05.2019 Bulletin 2019/20**

(51) Int Cl.:
***C11D 1/94*** (2006.01) ***C07K 14/415*** (2006.01)
***C11D 3/38*** (2006.01) ***C11D 11/00*** (2006.01)

(21) Application number: **18180838.7**

(22) Date of filing: **29.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.11.2017 EP 17201326**

(71) Applicant: **The Procter & Gamble Company**
**Cincinnati, OH 45202 (US)**

(72) Inventors:
- **BETTIOL, Jean-Luc Philippe**
  **1853 Strombeek-Bever (BE)**
- **GONZALES, Denis Alfred**
  **1853 Strombeek-Bever (BE)**
- **VELASQUEZ, Juan Esteban**
  **Cincinnati, Ohio 45202 (US)**
- **GEARY, Nicholas William**
  **Cincinnati, Ohio 45202 (US)**

(74) Representative: **Siddiquee, Sanaul Kabir**
**N.V. Procter & Gamble**
**Services Company S.A.**
**Temselaan 100**
**1853 Strombeek-Bever (BE)**

# (54) DETERGENT COMPOSITION COMPRISING MODIFIED SOY PROTEINS

(57) The present invention is directed to a detergent composition comprising a chemically or physically modified soy protein selected from the group consisting of a chemically or physically modified soy 2S conglycinin, and a chemically or physically modified soy 7S beta-conglycinin, a surfactant system, and optionally an unmodified soy protein selected from the group consisting of an unmodified soy 2S conglycinin, an unmodified soy 7S beta-conglycinin, and mixtures thereof. Methods of making and using such compositions are also provided.

EP 3 483 242 A1

## Description

REFERENCE TO A SEQUENCE LISTING

This application contains Sequence Listings in computer readable form. The computer readable form is incorporated herein by reference.

FIELD OF THE INVENTION

The present invention relates to a detergent composition comprising a chemically or physically modified soy protein selected from the group consisting of a chemically or physically modified soy 2S conglycinin, and a chemically or physically modified soy 7S beta-conglycinin, and a surfactant system. The composition provides one or more benefits, including good cleaning particularly good grease emulsification, long lasting suds especially in presence of greasy soils and surface modification that can contribute to second time cleaning benefits, improved drying, improved shine.

BACKGROUND OF THE INVENTION

Detergent compositions should provide good soil and/or grease cleaning while presenting a good suds profile in particular a long-lasting suds profile especially in the presence of greasy soils. Users usually see suds as an indicator of the performance of the detergent composition. Moreover, the user of a detergent composition may also use the suds profile and the appearance of the suds (e.g., density, whiteness) as an indicator that the wash solution still contains active cleaning ingredients. This is particularly the case for manual washing, also referred to herein as hand-washing, where the user usually doses the detergent composition depending on the suds remaining and renews the wash solution when the suds subsides or when the suds does not look thick enough. Thus, a detergent composition, particularly a manual wash detergent composition that generates little or low density suds would tend to be replaced by the user more frequently than is necessary. Thus, it is desirable for a detergent composition to provide "good sudsing profile", which includes good suds height and/or density as well as good suds duration during the initial mixing of the composition with water and/or during the entire washing operation.

Soy proteins are a family of proteins, hence consisting of long folded chains of amino acids. The inclusion of "unmodified" soy whey proteins as a sudsing agent in personal care and industrial products has been described in WO2014/018929. However, the inclusion of "modified" soy proteins has not been disclosed, particularly in the context of detergent compositions for improving sudsing profile. While unmodified proteins might positively impact the suds duration of a detergent composition even in presence of greasy soils, the Applicants believe that through modifying the proteins a more optimized affinity of the protein to emulsify greasy soils can be achieved, as such releasing the surfactants more from the oil-water interface and rendering them available to continue stabilizing the suds at the air water interface.

Accordingly, the need remains for an improved detergent composition comprising a chemically or physically modified soy protein which has a further improved sudsing profile, particularly at low chemically or physically modified soy protein concentrations in the detergent composition. The need also exists for an improved detergent composition, when used in a manual-washing process, the composition should also provide a pleasant washing experience, *i.e.*, good feel on the user's hands during the wash. The composition should also be easy to rinse. Further it is desirous that the improved detergent composition is stable and will not phase separate, resulting in greater shelf-life of the product. It is also desirable that detergent compositions provide surface modification, contributing to shine and/or improved second time cleaning. There is also the desire to reduce the amount of surfactants without negatively impacting sudsing nor grease cleaning and emulsification profile. Thus, there is the need to find new compositions that improve cleaning, suds longevity and improved after cleaning benefits in hand washing conditions. The Applicant discovered that some or all of the above-mentioned needs can be at least partially fulfilled through the improved detergent composition as described herein below.

SUMMARY OF THE INVENTION

The present invention meets one or more of these needs based on the surprising discovery that by formulating a detergent composition comprising a chemically or physically modified soy protein, a surfactant system, and optionally an unmodified soy protein, such a composition exhibits good sudsing profile, particularly desirable suds volume and/or sustained suds stabilization, especially in the presence of greasy soils. It also provides good grease cleaning and emulsification benefits and can also provide surface modifications facilitating next time cleaning benefit.

According to a first aspect, the present invention is directed to a detergent composition comprising: (i) a chemically or physically modified soy protein selected from the group consisting of: (a) from 20 wt% to 80 wt%, preferably from 30 wt% to 60 wt%, by weight of the modified soy protein, based on active proteins, of a chemically or physically

modified soy 2S conglycinin, preferably as modified by the procedures as shown in Table 2 of the description, and (b) from 20 wt% to 80 wt%, preferably from 40 wt% to 70 wt%, by weight of the modified soy protein of a chemically or physically modified soy 7S beta-conglycinin, and (ii) a surfactant system. The sum total wt% of the chemically or physically modified soy 2S conglycinin, and the chemically or physically modified soy 7S beta-conglycinin add up to 100 wt% by weight of the total chemically or physically modified soy protein, based on active protein. Preferably the detergent compositions comprises an unmodified soy protein preferably selected from the group consisting of an unmodified soy 2S conglycinin, an unmodified soy 7S beta-conglycinin, and mixtures thereof.

Preferably the detergent composition is a manual-washing composition. Preferably the detergent composition is for manual dishwashing. Preferred compositions are in the form of a liquid.

In another aspect, the present invention is directed to a method of manually washing dishware comprising the steps of delivering a detergent composition of the invention into a volume of water to form a wash solution and immersing the dishware in the solution.

In yet another aspect, there is provided a method of manual washing comprising the step of: delivering the detergent composition of the invention to a volume of water and immersing soiled articles in the water. When the composition of the invention is used according to this method a good sudsing profile, with a long lasting effect is achieved.

In yet another aspect, the present invention is directed to a method of manually washing dishware comprising the steps of: i) delivering a composition of the invention onto the dishware or a cleaning implement; ii) cleaning the dishware with the composition in the presence in water; and iii) optionally, rinsing the dishware.

In yet another aspect, the present invention relates to a method of manually washing dishware comprising: i) delivering a detergent composition according to the present invention onto the dishware or a cleaning implement; ii) cleaning the dishware with the composition in the presence of water; and iii) optionally, rinsing the dishware. Preferably, the composition of the present invention is used in neat form (*i.e.*, direct application) since greater benefits in terms of grease cleaning are obtained when the composition is directly applied on the soiled surface or on a cleaning implement, such as a sponge, to be used to clean the soiled surface.

There is also provided the use of a modified soy protein selected from the group consisting of: (a) a chemically or physically modified soy 2S conglycinin, preferably as modified by the procedures as shown in Table 2 of the description, preferably the modified soy 2S conglycinin is derived from an unmodified soy 2S conglycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of a native soy 2S conglycinin protein of SEQ ID NO: 1; and (b) a chemically or physically modified soy 7S beta-conglycinin, preferably as modified by the procedures as shown in Table 2 of the description, preferably the modified soy 7S beta-conglycinin is derived from an unmodified soy 7S beta-conglycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of at least one reference sequence selected from a native soy 7S beta-conglycinin selected from the group consisting of: SEQ ID NO: 2, SEQ ID NO: 3, and SEQ ID NO: 4, to provide increased suds longevity or increased grease emulsification in an aqueous wash liquor during a washing process.

Preferably the manual washing is dishwashing and the soiled articles comprise soiled dishware. As used herein, "dishware" includes cookware and tableware.

The elements of the composition of the invention described in relation to the first aspect of the invention apply *mutatis mutandis* to the other aspects of the invention.

These and other features, aspects and advantages of the present invention will become evident to those skilled in the art from the detailed description which follows.

## DETAILED DESCRIPTION OF THE INVENTION

### Definitions

As used herein, the articles "a" and "an" when used in a claim, are understood to mean one or more of what is claimed or described.

As used herein, the term "amino acid identity" means the identity between two or more amino acid sequences and is expressed in terms of the identity or similarity between the sequences. Sequence identity can be measured in terms of percentage identity; the higher the percentage, the more identical the sequences are. The percentage identity is calculated over the length of comparison. For example, the amino acid identity is typically calculated over the entire length of a sequence aligned against the entire length of the reference sequence (*e.g.*, SEQ ID NO: 1, SEQ ID NO: 2, SEQ ID NO: 3, SEQ ID NO: 4, SEQ ID NO: 5, or SEQ ID NO: 6). Methods of alignment of sequences for comparison are well known in the art and identity can be calculated by many known methods. Various programs and alignment

algorithms are described in the art. It should be noted that the terms 'sequence identity' and 'sequence similarity' can be used interchangeably.

As used herein, the term "detergent composition" refers to a composition or formulation designed for cleaning soiled surfaces. Such compositions include but are not limited to, dishwashing compositions, laundry detergent compositions, fabric softening compositions, fabric enhancing compositions, fabric freshening compositions, laundry pre-wash, laundry pretreat, laundry additives, spray products, dry cleaning agent or composition, laundry rinse additive, wash additive, post-rinse fabric treatment, ironing aid, hard surface cleaning compositions, unit dose formulation, delayed delivery formulation, detergent contained on or in a porous substrate or nonwoven sheet, and other suitable forms that may be apparent to one skilled in the art in view of the teachings herein. Such compositions may be used as a pre-cleaning treatment, a post-cleaning treatment, or may be added during the rinse or wash cycle of the cleaning process. The detergent compositions may have a form selected from liquid, powder, single-phase or multi-phase unit dose or pouch form, tablet, gel, paste, bar, or flake. Preferably the composition is for manual-washing. Preferably, the detergent composition of the present invention is a dishwashing detergent. Preferably the composition is in the form of a liquid.

As used herein the term "fragment" means an amino acid sequence of at least 30, 60, 100, 150 contiguous amino acids of the reference sequences or any integer there between.

As used herein the term "increased suds longevity" means an increase in the duration of visible suds in a washing process cleaning soiled articles using the composition comprising a modified soy protein, compared with the suds longevity provided by the same composition and process in the absence of the modified soy protein or relative to the unmodified soy protein.

As used herein, the term "modified soy protein" refers to a soy protein that has been chemically or physically modified. The term "chemically modified" soy protein in the context of the present invention is understood to mean soy proteins that are obtained by chemical reaction of the reactive groups of soy proteins. In particular the carboxyl, hydroxy, amine, imidazole, and/or thiol groups of the side chains of the amino acids of the soy protein. For example, chemically modified soy protein may include soy proteins that are partially or completely denatured, partially or completely desugarized, partially or completely esterified, partially hydrolyzed, or combinations thereof. The term "physically modified" soy protein in the context of the present invention is understood to mean soy proteins that are modified by physical effect, such as for example, by heat, light, fractionation, sonication, etc.

As used herein, the term "native" refers to a soy protein that is the wild-type sequence.

As used herein, the term "next time cleaning benefit" means the surface to be cleaned could be treated with a composition which would assist in easier removal of soil and/or stains during subsequent cleaning.

As used herein, the term "soiled surfaces" refers non-specifically to any type of flexible material consisting of a network of natural or artificial fibers, including natural, artificial, and synthetic fibers, such as, but not limited to, cotton, linen, wool, polyester, nylon, silk, acrylic, and the like, as well as various blends and combinations. Soiled surfaces may further refer to any type of hard surface, including natural, artificial, or synthetic surfaces, such as, but not limited to, tile, granite, grout, glass, composite, vinyl, hardwood, metal, cooking surfaces, plastic, and the like, as well as blends and combinations, as well as dishware. Key targeted soiled surfaces by this application are soiled dishware.

As used herein, the "unmodified soy protein" refers to a soy protein that has neither been treated by chemical nor physical methods.

As used herein, the term "variant" of the modified soy proteins means an amino acid sequence when the modified soy protein is modified by, or at, one or more amino acids (for example 1, 2, 3, 4, 5, 6, 7, 8, 9 or 10 or more amino acid modifications) selected from substitutions, insertions, deletions and combinations thereof. The variant may have "conservative" substitutions, wherein a substituted amino acid has similar structural or chemical properties to the amino acid that replaces it, for example, replacement of leucine with isoleucine. A variant may have "non-conservative" changes, for example, replacement of a glycine with a tryptophan. Variants may also include sequences with amino acid deletions or insertions, or both. Guidance in determining which amino acid residues may be substituted, inserted, or deleted without abolishing the activity of the protein may be found using computer programs well known in the art. Variants may also include truncated forms derived from a wild-type soy protein, such as for example, a soy protein with a truncated N-terminus.

As used herein, the term "water hardness" or "hardness" means uncomplexed cations ion (*i.e.*, $Ca^{2+}$ or $Mg^{2+}$) present in water that have the potential to precipitate under alkaline conditions, and thereby diminishing the surfactancy and cleaning capacity of surfactants. Further, the terms "high water hardness" and "elevated water hardness" can be used interchangeably and are relative terms for the purposes of the present invention, and are intended to include, but not limited to, a hardness level containing at least 12 grams of calcium ion per gallon water (gpg, "American grain hardness" units).

## Detergent Composition

A preferred detergent composition is a manual dishwashing composition, preferably in liquid form. It typically

contains from 30% to 95%, preferably from 40% to 90%, more preferably from 50% to 85% by weight of the composition of a liquid carrier in which the other essential and optional components are dissolved, dispersed or suspended. One preferred component of the liquid carrier is water.

Preferably the pH of the composition, measured as a 10% product concentration in demineralized water at 20°C, is adjusted to between 3 and 14, more preferably between 4 and 13, more preferably between 6 and 12 and most preferably between 8 and 10. The pH of the composition can be adjusted using pH modifying ingredients known in the art.

Soy Proteins

2S conglycinin is a small storage, highly charged, helix rich protein with 138 amino acid residues and a molecular weight of 18.4 kDa. 7S beta-conglycinin is an aggregate of 3 subunits selected from the group of $\alpha$-beta-conglycinin (with molecular weight of 70.7 kDa and 605 amino acid residues), $\alpha$'-beta-conglycinin (with molecular weight of 74.3 kDa and 639 amino-acid residues) and $\beta$-beta-conglycinin (with molecular weight of 50.5 kDa and 439 amino-acid residues). 7S beta-conglycinin is a glycol-protein with an isoelectrical point of 4.5-5.5 with little/no disulfide bonds which allow effective structural modification during the absorption at the interface. 11S glycinin is an aggregate of 6 units having a molecular weight close to 58 kDa and 516 amino-acid residues with internal disulfide bond which limits its ability to absorb effectively at the interface.

Unexpectedly, the Applicants found that a "modified" soy protein, in particular a modified soy protein selected from the group consisting of a chemically or physically modified soy 2S conglycinin, and a chemically or physically modified soy 7S beta-conglycinin, is able to produce a more stable hence longer lasting sudsing profile in detergent wash solutions comprising oily and/or greasy soils, compared to the unmodified soy protein. Not wishing to be bound by theory, the Applicants believe that the increased sudsing benefits are due to differences in amino acid sequences and/or protein structures of the modified versus the unmodified soy proteins. The modified soy protein has improved oil and grease affinity hence oil and grease emulsification properties, and as such releasing surfactant molecules otherwise consumed for oil emulsification to continue stabilizing suds throughout the wash process. It is believed that any of the modified soy protein absorb at the interface, undergo structural arrangement to ensure a most favorable conformation of the hydrophobic amino-acid residues and form a complex network at the interface. It is likely that each of the three modified soy proteins undergo various absorption kinetics or mechanisms hence the use of blend is especially preferred.

While unmodified soy proteins are recognized to improve suds stability, the performance at forming high quality suds is greatly affected by their capacity to expose hydrophobic amino-acid residues to the interface both kinetically and quantitatively. In practice, unmodified soy protein have most of their hydrophobic amino-acid residues folded inward of the protein structures, which together with some lack of structural flexibility, *e.g.*, optionally due to the presence of disulfide bonds, prevents the soy protein to quickly or efficiently expose its hydrophobic amino-acid residues at the interface. At time, this is also prevented or slowed down by the aggregation of soy proteins and/or soy protein sub-units.

The intent of the soy protein modification is fully linked to the desire to have de-aggregated soy protein with any of the following attribute, *e.g.*, more flexible soy protein, molten globular or molten folded soy protein, that can achieve more quickly and/or more complete exposure hydrophobic residues, alternatively partially or fully unfolded, extended soy protein, alternatively residues from partial hydrolysis of the soy proteins, alternatively the incorporation of more hydrophobic residues by chemical modification, *e.g.*, esterification.

Accordingly, a detergent composition of the present invention comprises a chemically or physically modified soy protein selected from the group consisting of a chemically or physically modified soy 2S conglycinin, and a chemically or physically modified soy 7S beta-conglycinin. Preferably the chemically or physically modified soy 2S conglycinin is derived from an unmodified soy 2S conglycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of a native soy 2S conglycinin protein of SEQ ID NO: 1. Preferably the chemically or physically modified soy 7S beta-conglycinin is derived from an unmodified soy 7S beta-conglycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of a native soy 7S beta-conglycinin selected from the group consisting of: SEQ ID NO: 2, SEQ ID NO: 3, and SEQ ID NO: 4.

The detergent composition of the invention comprises a chemically or physically modified soy protein wherein the chemically or physically modified soy protein comprises:

a) from 20 wt% to 80 wt%, preferably from 40 wt% to 60 wt%, by weight of the total chemically or physically modified soy protein, based on active protein, of a chemically or physically modified soy 2S conglycinin; and
b) from 20 wt% to 80 wt%, preferably from 40 wt% to 60 wt%, by weight of the total chemically or physically modified soy protein, based on active protein, of a chemically or physically modified soy 7S beta-conglycinin.

Preferably, the detergent composition of the invention further comprises an unmodified soy protein preferably selected from the group consisting of an unmodified soy 2S conglycinin, an unmodified soy 7S beta-conglycinin, and mixtures thereof. Preferably the unmodified soy 2S conglycinin is the remaining unmodified portion of the unmodified soy 2S conglycinin used to generate the chemically or physically modified soy 2S conglycinin from above, and wherein the unmodified 7S beta-conglycinin is the remaining unmodified portion of the unmodified soy 7S beta-conglycinin used to generate the chemically or physically modified soy 7S beta-conglycinin from above.

Preferably the unmodified soy 2S conglycinin has at least at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of a native soy 2S conglycinin protein of SEQ ID NO: 1. Preferably the unmodified soy 7S beta-conglycinin has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of a native soy 7S beta-conglycinin selected from the group consisting of: SEQ ID NO: 2, SEQ ID NO: 3, and SEQ ID NO: 4.

Preferably, a detergent composition of the present invention wherein the chemically or physically modified soy protein further comprises a chemically or physically modified soy 11S glycinin. Accordingly, the detergent composition comprises:

(i) a chemically or physically modified soy protein selected from the group consisting of:

(a) from 6 wt% to 56 wt%, preferably from 12 wt% to 36 wt%, by weight of the total chemically or physically modified soy protein, based on active protein, of a chemically or physically modified soy 2S conglycinin, preferably as modified by the procedures as shown in Table 2 of the description;

(b) from 6 wt% to 56 wt%, preferably from 16 wt% to 42 wt%, by weight of the total chemically or physically modified soy protein, based on active protein, of a chemically or physically modified soy 7S beta-conglycinin, preferably as modified by the procedures as shown in Table 2 of the description; and

(c) from 30 wt% to 70 wt%, preferably from 40 wt% to 60 wt%, by weight of the total chemically or physically modified soy protein, based on active protein, a chemically or physically modified soy 11S glycinin, preferably as modified by the procedures as shown in Table 2 of the description;

wherein the sum total wt% of the chemically or physically modified soy 2S conglycinin, the chemically or physically modified soy 7S beta-conglycinin, and the chemically or physically modified soy 11S glycinin add up to 100 wt% by weight of the total chemically or physically modified soy protein, based on active protein; and

(ii) a surfactant system.

Preferably the chemically or physically modified soy 11S glycinin is derived from an unmodified soy 11S glycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of at least one reference sequence selected from a native soy 11S glycinin protein selected from the group consisting of SEQ ID NO: 5, and SEQ ID NO: 6.

Preferably the detergent composition further comprises an unmodified soy 11S glycinin, wherein the unmodified soy 11S glycinin is the remaining unmodified portion of the unmodified soy 11S glycinin used to generate the chemically or physically modified soy 11S glycinin from above.

Preferably, the detergent composition of the present invention wherein the chemically or physically modified soy protein further comprises a chemically or physically modified soy 15S glycinin and a chemically or physically modified soy 11S glycinin. Accordingly, the detergent composition comprises:

(i) a chemically or physically modified soy protein selected from the group consisting of;

(a) from 5 wt% to 55 wt%, preferably from 11 wt% to 35 wt% by weight of the total chemically or physically modified soy protein, based on active protein, of a chemically or physically modified soy 2S conglycinin, preferably as modified by the procedures as shown in Table 2 of the description;

(b) from 5 wt% to 55 wt%, preferably from 15 wt% to 41 wt%, by weight of the total chemically or physically modified soy protein, based on active protein, of a chemically or physically modified soy 7S beta-conglycinin, preferably as modified by the procedures as shown in Table 2 of the description;

(c) from 24 wt% to 69 wt%, preferably from 36 wt% to 59 wt% by weight of the total chemically or physically modified soy protein, based on active protein, of a chemically or physically modified soy 11S glycinin, preferably as modified by the procedures as shown in Table 2 of the description; and

(d) from 1 wt% to 20 wt%, preferably from 1 wt% to 10 wt%, by weight of the total chemically or physically modified soy protein, based on active protein, of the chemically or physically modified soy 15S glycinin, preferably as modified by the procedures as shown in Table 2 of the description;

wherein the sum total wt% of the chemically or physically modified soy 2S conglycinin, the chemically or physically modified soy 7S beta-conglycinin, the chemically or physically modified soy 11S glycinin, and the chemically or physically modified soy 15S glycinin add up to 100 wt%, by weight of the total chemically or physically modified soy protein, based on active protein; and

(ii) a surfactant system.

Preferably the chemically or physically modified soy 15S glycinin is derived from an unmodified soy 15S glycinin which is a dimer of an unmodified soy 11S glycinin, as described above (Wolf et al., J. Agric. Good. Chem., 1996, 44, pp. 785-791). Preferably, the chemically or physically modified soy 15S glycinin is derived from an unmodified soy 15S glycinin which is a dimer of an unmodified soy 11S glycinin, wherein the unmodified soy 11S glycinin monomers have at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of at least one reference sequence selected from a native soy 11S glycinin protein selected from the group consisting of SEQ ID NO: 5, and SEQ ID NO: 6.

Preferably the detergent composition further comprises an unmodified soy 15S glycinin, wherein the unmodified soy 15S glycinin is the remaining unmodified portion of the unmodified soy 15S glycinin used to generate the chemically or physically modified soy 15S glycinin from above.

Identity, or homology, percentages as mentioned herein in respect of the present invention are those that can be calculated with the GAP program, obtainable from GCG (Genetics Computer Group Inc., Madison, WI, USA). Alternatively, a manual alignment can be performed.

For polypeptide sequence comparison the following settings can be used: Alignment algorithm: Needleman and Wunsch, J. Mol. Biol. 1970, 48: 443-453. As a comparison matrix for amino acid similarity the Blosum62 matrix is used (Henikoff S. and Henikoff J.G., P.N.A.S. USA 1992, 89: 10915-10919). The following gap scoring parameters are used: Gap penalty: 12, gap length penalty: 2, no penalty for end gaps. A given sequence is typically compared against the full-length sequence of SEQ ID NO: 1, SEQ ID NO: 2, SEQ ID NO: 3, SEQ ID NO: 4, SEQ ID NO: 5, or SEQ ID NO: 6, to obtain a score.

Preferably the detergent composition of the invention comprises a chemically or physically modified soy protein wherein the chemically or physically modified soy protein is present in an amount of from 0.01 wt% to 5 wt%, preferably from 0.1% to 2.5 wt%, by weight of the composition, based on active protein.

The invention also includes variants in the form of truncated forms derived from a wild-type chemically or physically modified soy protein, such as a soy protein with a truncated N-terminus. The chemically or physically modified soy protein is predicted to include an N-terminal signal peptide that is likely removed upon secretion by the native organisms. The current invention may also include variants without the N-terminal signal peptide. For example, a chemically or physically modified soy variant protein derived from a native soy variant protein which corresponds to the sequence of full length wild-type native soy 2S conglycinin protein (SEQ ID NO: 1) without the predicted N-terminal signal peptide, is also part of the current invention. Bioinformatic tools, such as for example, signal peptide prediction server SignalP version 4.1 (Petersen TN., Brunak S., von Heijne G. and Nielsen H. (2011). Nature Methods, 8:785-786), can be used to predict the existence and length of such signal peptides.

It is important that variants of chemically or physically modified soy proteins retain and preferably improve the ability of the wild-type proteins to adsorb at an interface and to stabilize that interface. Some performance drop in a given property of chemically or physically modified soy protein variants may of course be tolerated, but the chemically or physically modified soy protein variants should retain and preferably improve suitable properties for the relevant application for which they are intended. For instance, screening of variants of one of the wild-types can be used to identify whether they retain and preferably improve appropriate properties.

Suitable examples of chemically or physically modified soy protein variants are derived from unmodified soy protein variants which include one conservative substitution in the peptide, such as a conservative substitution in SEQ ID NO: 1, SEQ ID NO: 2, SEQ ID NO: 3, or SEQ ID NO: 4, SEQ ID NO: 5, or SEQ ID NO: 6.

Other suitable examples of chemically or physically modified soy protein variants are derived from unmodified soy protein variants which include 10 or fewer conservative substitutions in the peptide, such as five or fewer. The chemically or physically modified soy proteins of the invention may therefore be derived from unmodified soy protein variants which include 1, 2, 3, 4, 5, 6, 7, 8, 9, 10 or more conservative substitutions. The chemically or physically modified soy proteins can be derived from unmodified soy proteins which can be produced to contain one or more conservative substitutions by manipulating the nucleotide sequence that encodes them using, for example, standard procedures such as site-directed mutagenesis or PCR. Alternatively, the chemically or physically modified soy proteins can be derived

from unmodified soy proteins which can be produced to contain one or more conservative substitutions by using peptide synthesis methods, for example, as known in the art.

Examples of amino acids which may be substituted for an original amino acid in the unmodified soy protein and which are regarded as conservative substitutions include: Ser for Ala; Lys for Arg; Gln or His for Asn; Glu for Asp; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

The chemically or physically modified soy proteins of the invention may be derived from unmodified soy proteins which comprise variants of SEQ ID NO: 1, SEQ ID NO: 2, SEQ ID NO: 3, or SEQ ID NO: 4, SEQ ID NO: 5, or SEQ ID NO: 6, wherein a short amino acid sequence containing two cysteine residues is added at the C-terminus. These cysteine residues can allow the modified soy protein variants to form multimers (*i.e.*, dimers, tetramers, hexamers and potentially higher order oligomers) in solution due to the formation of disulfide bonds between the cysteine residues of adjacent modified soy protein variants.

The chemically or physically modified soy proteins of the invention can also be derived from unmodified soy proteins which may also cover any fragments of SEQ ID NO: 1, SEQ ID NO: 2, SEQ ID NO: 3, or SEQ ID NO: 4, SEQ ID NO: 5, or SEQ ID NO: 6. Preferably the chemically or physically modified soy protein fragments can adsorb to an interface and stabilize that interface.

Preferably the modified soy proteins are chemically modified, physically modified or combinations thereof. Accordingly the chemically modified soy proteins of the invention are chemically modified by a variety of chemical techniques to produce derivatives having essentially the same or preferably improved activity as the unmodified peptides, and optionally having other desirable properties. For example, carboxylic acid groups of the protein, whether carboxyl-terminal or side chain, may be provided in the form of a salt of a pharmaceutically-acceptable cation or esterified, for example to form a C1-C6 alkyl ester, or converted to an amide, for example of formula $CONR_1R_2$ wherein $R_1$ and $R_2$ are each independently H or C1-C6 alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C1-C6 alkyl or dialkyl amino or further converted to an amide. Hydroxyl groups of the peptide side chains may be converted to alkoxy or ester groups, for example C1-C6 alkoxy or C1-C6 alkyl ester, using well-recognized techniques. Phenyl and phenolic rings of the peptide side chains may be substituted with one or more halogen atoms, such as F, Cl, Br or I, or with C1-C6 alkyl, C1-C6 alkoxy, carboxylic acids and esters thereof, or amides of such carboxylic acids. Methylene groups of the peptide side chains can be extended to homologous C2-C4 alkylenes. Thiols can be protected with any one of a number of well-recognized protecting groups, such as acetamide groups.

Those skilled in the art will also recognize methods for introducing cyclic structures into the modified soy proteins of the present invention to select and provide conformational constraints to the structure that result in enhanced stability.

Preferably the chemically or physically modified soy protein is a partially or completely denatured protein. Preferably the denatured soy protein has been denatured by:

i) application of heat to the soy protein at a temperature of from 60°C to 100°C;
ii) pH treatment of the soy protein to a pH of from 1 to 4 or from 9 to 13;
iii) chemical denaturation of the soy protein;
iv) subjecting the soy protein to sonication, preferably high frequency sonication, in a range of from 5 kHz to 50 kHz; or
v) combinations of i) to iv).

Preferably the chemically or physically modified soy protein is derived from partial or complete desugarization of the soy protein, preferably the desugarization being performed with enzyme, bacteria or yeast, preferably by *S. cerevisiae.*

Preferably the chemically or physically modified soy protein is derived from partial or complete esterification of the soy protein with C1-C12 linear, cyclic or aromatic alcohol, or with chloroformate. Preferably the esterification is being performed with chloroformate, more preferably phenyl chloroformate optionally blended with phenol.

Preferably the chemically or physically modified soy protein is a partially hydrolyzed soy protein, preferably derived from partial hydrolysis of the soy protein. Preferably the partial hydrolysis being performed enzymatically by protease and the degree of hydrolyzation is 50% or less, preferably 20% or less, more preferably 10% or less, more preferably the protease is papain.

Preferably the chemically or physically modified soy protein is a modified soy 2S conglycinin, and a modified 7S beta-conglycinin, preferably a soy 2S conglycinin hydrolizates, and a 7S beta-conglycinin hydrolizates. The modified soy protein may comprise one or more subunits. Preferably each of the subunit of the modified soy protein has a weight average molecular weight of 2,000 kDa to 75,000 kDa. The weight average molecular weight is determined by the specific ASTM method for each polymer, but is generally measured using either gel permeation chromatography (GPC) or from solution viscosity measurements. The thermoplastic polymer weight average molecular weight should be determined before addition into the admixture.

Preferably the soy 7S beta-conglycinin is a blend selected from the group consisting of soy 7S beta-conglycinin alpha chain, soy 7S beta-conglycinin alpha' chain, soy 7S beta-conglycinin beta chain, and mixtures thereof.

Preferably the pH of the composition is from 7 to 10, preferably from 8.5 to 9, when measured as a 10% product concentration in demineralized water at 20°C.

Surfactant System

While the protein can also be formulated in absence of a surfactant, the detergent composition preferably also comprises a surfactant system. Preferably the detergent composition comprises from 1% to 60%, preferably from 5% to 50%, more preferably from 8% to 40%, by weight of the total composition of a surfactant system.

The surfactant system of the composition of the present invention comprises an anionic surfactant. Preferably, the surfactant system for the cleaning composition of the present invention comprises from 1% to 40%, preferably 6% to 35%, more preferably 8% to 30% by weight of the total composition of an anionic surfactant. The anionic surfactant can be any anionic cleaning surfactant, preferably selected from sulfate and/or sulfonate anionic surfactants. HLAS (linear alkylbenzene sulfonate) would be the most preferred sulfonate anionic surfactant. Especially preferred anionic surfactant is selected from the group consisting of alkyl sulfate, alkyl alkoxy sufate and mixtures thereof, and preferably wherein the alkyl alkoxy sulfate is an alkyl ethoxy sulfate. Preferred anionic surfactant is a combination of alkyl sulfates and alkyl ethoxy sulfates with a combined average ethoxylation degree of less than 5, preferably less than 3, more preferably less than 2 and more than 0.5 and an average level of branching of from about 5% to about 40%, more preferably from about 10% to 35%, and even more preferably from about 20% to 30%.

The average alkoxylation degree is the mol average alkoxylation degree of all the components of the mixture (*i.e.*, mol average alkoxylation degree) of the anionic surfactant. In the mol average alkoxylation degree calculation the weight of sulfate anionic surfactant components not having alkoxylate groups should also be included.

$$\text{Mol average alkoxylation degree} = (x1 * \text{alkoxylation degree of surfactant } 1 + x2 * \text{alkoxylation degree of surfactant } 2 + \ldots) / (x1 + x2 + \ldots)$$

wherein x1, x2, ... are the number of moles of each sulfate anionic surfactant of the mixture and alkoxylation degree is the number of alkoxy groups in each sulfate anionic surfactant.

The average level of branching is the weight average % of branching and it is defined according to the following formula:

$$\text{Weight average of branching (\%)} = [(x1 * \text{wt\% branched alcohol } 1 \text{ in alcohol } 1 + x2 * \text{wt\% branched alcohol } 2 \text{ in alcohol } 2 + \ldots) / (x1 + x2 + \ldots)] * 100$$

wherein x1, x2, ... are the weight in grams of each alcohol in the total alcohol mixture of the alcohols which were used as starting material for the anionic surfactant for the composition of the invention. In the weight average branching degree calculation the weight of anionic surfactant components not having branched groups should also be included.

Suitable examples of commercially available sulfates include, those based on Neodol alcohols ex the Shell company, Lial - Isalchem and Safol ex the Sasol company, natural alcohols ex The Procter & Gamble Chemicals company. Suitable sulfonate surfactants for use herein include water-soluble salts of C8-C18 alkyl or hydroxyalkyl sulfonates; C11-C18 alkyl benzene sulfonates (LAS), modified alkylbenzene sulfonate (MLAS); methyl ester sulfonate (MES); and alpha-olefin sulfonate (AOS). Those also include the paraffin sulfonates may be monosulfonates and/or disulfonates, obtained by sulfonating paraffins of 10 to 20 carbon atoms. The sulfonate surfactant also include the alkyl glyceryl sulfonate surfactants.

The surfactant system of the composition of the present invention further comprises a primary co-surfactant system, wherein the primary co-surfactant system is preferably selected from the group consisting of amphoteric surfactant, zwitterionic surfactant and mixtures thereof. Preferably, the surfactant system for the cleaning composition of the present invention comprises from 0.5% to 15%, preferably from 1% to 12%, more preferably from 2% to 10%, by weight of the total composition of a primary co-surfactant system.

Preferably the primary co-surfactant system is an amphoteric surfactant. Preferably, the primary co-surfactant system is an amine oxide surfactant, and wherein the composition comprises anionic surfactant and amine oxide surfactant in a ratio of less than 9:1, more preferably from 5:1 to 1:1, more preferably from 4:1 to 2:1, preferably from 3:1

to 2.5:1. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or mid-branched alkyl moiety. Typical linear amine oxides include water-soluble amine oxides containing one R1 C8-18 alkyl moiety and 2 R2 and R3 moieties selected from the group consisting of C1-3 alkyl groups and C1-3 hydroxyalkyl groups. Preferably amine oxide is characterized by the formula R1 - N(R2)(R3) O wherein R1 is a C8-18 alkyl and R2 and R3 are selected from the group consisting of methyl, ethyl, propyl, isopropyl, 2-hydroxethyl, 2-hydroxypropyl and 3-hydroxypropyl. The linear amine oxide surfactants in particular may include linear C10-C18 alkyl dimethyl amine oxides and linear C8-C12 alkoxy ethyl dihydroxy ethyl amine oxides. Preferred amine oxides include linear C10, linear C10-C12, and linear C12-C14 alkyl dimethyl amine oxides. As used herein "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 is from 10 to 24 carbon atoms, preferably from 12 to 20, and more preferably from 10 to 16. The number of carbon atoms for the one alkyl moiety (n1) should be approximately the same number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a C1-3 alkyl, a C1-3 hydroxyalkyl group, or a polyethylene oxide group containing an average of from about 1 to about 3 ethylene oxide groups. Preferably, the two moieties are selected from a C1-3 alkyl, more preferably both are selected as a C1 alkyl.

Preferably the amine oxide surfactant is a mixture of amine oxides comprising a low-cut amine oxide and a mid-cut amine oxide. The amine oxide of the composition of the invention then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls or mixtures thereof; and
b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl. In another preferred low-cut amine oxide for use herein R1 and R2 are both methyl. In an especially preferred low-cut amine oxide for use herein R1 and R2 are both methyl and R3 is n-decyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Compositions comprising R7R8R9AO tend to be unstable and do not provide very suds mileage.

Preferably the primary co-surfactant system is a zwitterionic surfactant. Suitable exampes of zwitterionic surfactants include betaines, such as alkyl betaines, alkylamidobetaine, amidazoliniumbetaine, sulfobetaine (INCI Sultaines) as well as the Phosphobetaine and preferably meets formula (I):

R1-[CO-X (CH2)n]x-N+(R2)(R3)-(CH2)m-[CH(OH)-CH2]y-Y- (I)

wherein:

R1 is a saturated or unsaturated C6-22 alkyl residue, preferably C8-18 alkyl residue, in particular a saturated C10-16 alkyl residue, for example a saturated C12-14 alkyl residue;
X is NH, NR4 with CI-4 Alkyl residue R4, O or S;
n is a number from 1 to 10, preferably 2 to 5, in particular 3;
x is 0 or 1, preferably 1;
R2 and R3 are independently a C1-4 alkyl residue, potentially hydroxy substituted such as a hydroxyethyl, preferably a methyl;
m is a number from 1 to 4, in particular 1, 2 or 3;
y is 0 or 1; and
Y is COO, SO3, OPO(OR5)O or P(O)(OR5)O, whereby R5 is a hydrogen atom H or a C1-4 alkyl residue.

Preferred betaines are the alkyl betaines of the formula (Ia), the alkyl amido propyl betaine of the formula (Ib), the Sulfo betaines of the formula (Ic), and the Amido sulfobetaine of the formula (Id);

R1-N+(CH3)2-CH2COO- (Ia)

R1-CO-NH(CH2)3-N+(CH3)2-CH2COO- (Ib)

R1-N+(CH3)2-CH2CH(OH)CH2SO3- (Ic)

R1-CO-NH-(CH2)3-N+(CH3)2-CH2CH(OH)CH2SO3- (Id)

in which R11 as the same meaning as in formula I. Particularly preferred betaines are the Carbobetaine [wherein Y-=COO-], in particular the Carbobetaine of the formula (Ia) and (Ib), more preferred are the Alkylamidobetaine of the formula (Ib).

Examples of suitable betaines and sulfobetaine are the following [designated in accordance with INCI]: Almondamidopropyl of betaines, Apricotam idopropyl betaines, Avocadamidopropyl of betaines, Babassuamidopropyl of betaines, Behenam idopropyl betaines, Behenyl of betaines, betaines, Canolam idopropyl betaines, Capryl/Capram idopropyl betaines, Carnitine, Cetyl of betaines, Cocamidoethyl of betaines, Cocam idopropyl betaines, Cocam idopropyl Hydroxysultaine, Coco betaines, Coco Hydroxysultaine, Coco/Oleam idopropyl betaines, Coco Sultaine, Decyl of betaines, Dihydroxyethyl Oleyl Glycinate, Dihydroxyethyl Soy Glycinate, Dihydroxyethyl Stearyl Glycinate, Dihydroxyethyl Tallow Glycinate, Dimethicone Propyl of PG-betaines, Erucam idopropyl Hydroxysultaine, Hydrogenated Tallow of betaines, Isostearam idopropyl betaines, Lauram idopropyl betaines, Lauryl of betaines, Lauryl Hydroxysultaine, Lauryl Sultaine, Milkam idopropyl betaines, Minkamidopropyl of betaines, Myristam idopropyl betaines, Myristyl of betaines, Oleam idopropyl betaines, Oleam idopropyl Hydroxysultaine, Oleyl of betaines, Olivamidopropyl of betaines, Palmam idopropyl betaines, Palm itam idopropyl betaines, Palmitoyl Carnitine, Palm Kernelam idopropyl betaines, Polytetrafluoroethylene Acetoxypropyl of betaines, Ricinoleam idopropyl betaines, Sesam idopropyl betaines, Soyam idopropyl betaines, Stearam idopropyl betaines, Stearyl of betaines, Tallowam idopropyl betaines, Tallowam idopropyl Hydroxysultaine, Tallow of betaines, Tallow Dihydroxyethyl of betaines, Undecylenam idopropyl betaines and Wheat Germam idopropyl betaines. A preferred betaine is, for example, Cocoamidopropylbetaine.

Preferably, the surfactant system of the composition of the present invention further comprises from 0.1% to 10% by weight of the total composition of a secondary co-surfactant system preferably comprising a non-ionic surfactant. Suitable non-ionic surfactants include the condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 18 carbon atoms, preferably from 10 to 15 carbon atoms with from 2 to 18 moles, preferably 2 to 15, more preferably 5-12 of ethylene oxide per mole of alcohol. Highly preferred non-ionic surfactants are the condensation products of guerbet alcohols with from 2 to 18 moles, preferably 2 to 15, more preferably 5-12 of ethylene oxide per mole of alcohol. Preferably, the non-ionic surfactants are an alkyl ethoxylated surfactants, preferably comprising from 9 to 15 carbon atoms in its alkyl chain and from 5 to 12 units of ethylene oxide per mole of alcohol. Other suitable non-ionic surfactants for use herein include fatty alcohol polyglycol ethers, alkylpolyglucosides and fatty acid glucamides, preferably alkylpolyglucosides. Preferably the alkyl polyglucoside surfactant is a C8-C16 alkyl polyglucoside surfactant, preferably a C8-C14 alkyl polyglucoside surfactant, preferably with an average degree of polymerization of between 0.1 and 3, more preferably between 0.5 and 2.5, even more preferably between 1 and 2. Most preferably the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6. C8-C16 alkyl polyglucosides are commercially available from several suppliers (*e.g.*, Simusol® surfactants from Seppic Corporation; and Glucopon® 600 CSUP, Glucopon® 650 EC, Glucopon® 600 CSUP/MB, and Glucopon® 650 EC/MB, from BASF Corporation). Preferably, the composition comprises the anionic surfactant and the non-ionic surfactant in a ratio of from 2:1 to 50:1, preferably 2:1 to 10:1.

Enzymes

Preferred compositions of the invention may comprise one or more enzymes selected from the group consisting of amylases, lipases, proteases, cellulases, lipoxygenases, diol synthases, and mixtures thereof. Each additional enzyme is typically present in an amount from 0.0001 wt% to 1 wt%, preferably from 0.0005 wt% to 0.5 wt%, more preferably 0.005 wt% to 0.1 wt%, by weight of the composition, based on active protein.

Enzyme Stabilizer

When comprising enzymes preferably the composition of the invention comprises an enzyme stabilizer. Suitable enzyme stabilizers may be selected from the group consisting of (a) univalent, bivalent and/or trivalent cations preferably

selected from the group of inorganic or organic salts of alkaline earth metals, alkali metals, aluminum, iron, copper and zinc, preferably alkali metals and alkaline earth metals, preferably alkali metal and alkaline earth metal salts with halides, sulfates, sulfites, carbonates, hydrogencarbonates, nitrates, nitrites, phosphates, formates, acetates, propionates, citrates, maleates, tartrates, succinates, oxalates, lactates, and mixtures thereof. In a preferred embodiment the salt is selected from the group consisting of sodium chloride, calcium chloride, potassium chloride, sodium sulfate, potassium sulfate, sodium acetate, potassium acetate, sodium formate, potassium formate, calcium lactate, calcium nitrate and mixtures thereof. Most preferred are salts selected from the group consisting of calcium chloride, potassium chloride, potassium sulfate, sodium acetate, potassium acetate, sodium formate, potassium formate, calcium lactate, calcium nitrate, and mixtures thereof, and in particular potassium salts selected from the group of potassium chloride, potassium sulfate, potassium acetate, potassium formate, potassium propionate, potassium lactate and mixtures thereof. Most preferred are potassium acetate and potassium chloride. Preferred calcium salts are calcium formate, calcium lactate and calcium nitrate including calcium nitrate tetrahydrate. Calcium and sodium formate salts may be preferred. These cations are present at at least 0.01 wt%, preferably at least 0.03 wt%, more preferably at least 0.05 wt%, most preferably at least 0.25 wt% up to 2 wt% or even up to 1 wt% by weight of the total composition. These salts are formulated from 0.1 to 5 wt%, preferably from 0.2 to 4 wt%, more preferably from 0.3 to 3 wt%, most preferably from 0.5 to 2 wt% relative to the total weight of the composition. Further enzyme stabilizers can be selected from the group (b) carbohydrates selected from the group consisting of oligosaccharides, polysaccharides and mixtures thereof, such as a monosaccharide glycerate as described in WO201219844; (c) mass efficient reversible protease inhibitors selected from the group consisting of phenyl boronic acid and derivatives thereof, preferably 4-formyl phenylboronic acid; (d) alcohols such as 1,2-propane diol, propylene glycol; (e) peptide aldehyde stabilizers such as tripeptide aldehydes such as Cbz-Gly-Ala-Tyr-H, or disubstituted alaninamide; (f) carboxylic acids such as phenyl alkyl dicarboxylic acid as described in WO2012/19849 or multiply substituted benzyl carboxylic acid comprising a carboxyl group on at least two carbon atoms of the benzyl radical such as described in WO2012/19848, phthaloyl glutamine acid, phthaloyl asparagine acid, aminophthalic acid and/or an oligoamino-biphenyl-oligocarboxylic acid; and (g) mixtures thereof.

Salt

The composition of the present invention may optionally comprise from 0.01% to 3%, preferably from 0.05% to 2%, more preferably from 0.2% to 1.5%, or most preferably 0.5% to 1%, by weight of the total composition of a salt, preferably a monovalent, divalent inorganic salt or a mixture thereof, preferably sodium chloride. Most preferably the composition alternatively or further comprises a multivalent metal cation in the amount of from 0.01 wt% to 2 wt%, preferably from 0.1% to 1%, more preferably from 0.2% to 0.8% by weight of the composition, preferably the multivalent metal cation is magnesium, aluminium, copper, calcium or iron, more preferably magnesium, most preferably said multivalent salt is magnesium chloride. Without wishing to be bound by theory, it is believed that use of a multivalent cation helps with the formation of protein/ protein, surfactant/ surfactant or hybrid protein/ surfactant network at the oil water and air water interface that is strengthening the suds.

Carbohydrates

Preferably the composition of the present invention comprises one or more carbohydrates selected from the group comprising O-glycan, N-glycan, and mixtures thereof. Suitable carbohydrates include alpha or beta glucan with 1,3 and/or 1.4 and/or 1,6 linkage. Glucans can be modified especially with carboxyl sulfate, glycol ether of amino groups. Glucan can be extracted from dextran, starch or cellulose. Glucan with structure close to natural glucan such as schizophyllan, scleroglucan or paramylon are particularly preferred.

Hydrotrope

The composition of the present invention may optionally comprise from 1% to 10%, or preferably from 0.5% to 10%, more preferably from 1% to 6%, or most preferably from 0.1% to 3%, or combinations thereof, by weight of the total composition of a hydrotrope, preferably sodium cumene sulfonate. Other suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903. Preferably the composition of the present invention is isotropic. An isotropic composition is distinguished from oil-in-water emulsions and lamellar phase compositions. Polarized light microscopy can assess whether the composition is isotropic. See *e.g*., The Aqueous Phase Behaviour of Surfactants, Robert Laughlin, Academic Press, 1994, pp. 538-542. Preferably an isotropic composition is provided. Preferably the composition comprises 0.1% to 3% by weight of the total composition of a hydrotrope, preferably wherein the hydrotrope is selected from sodium, potassium, and ammonium xylene sulfonate, sodium, potassium and ammonium toluene sulfonate, sodium potassium

and ammonium cumene sulfonate, and mixtures thereof.

Organic Solvent

The composition of the present invention may optionally comprise an organic solvent. Suitable organic solvents include C4-14 ethers and diethers, polyols, glycols, alkoxylated glycols, C6-C16 glycol ethers, alkoxylated aromatic alcohols, aromatic alcohols, aliphatic linear or branched alcohols, alkoxylated aliphatic linear or branched alcohols, alkoxylated C1-C5 alcohols, C8-C14 alkyl and cycloalkyl hydrocarbons and halohydrocarbons, and mixtures thereof. Preferably the organic solvents include alcohols, glycols, and glycol ethers, alternatively alcohols and glycols. The composition comprises from 0% to less than 50%, preferably from 0.01% to 25%, more preferably from 0.1% to 10%, or most preferably from 0.5% to 5%, by weight of the total composition of an organic solvent, preferably an alcohol, more preferably an ethanol, a polyalkyleneglycol, more preferably polypropyleneglycol, and mixtures thereof.

Amphiphilic Polymer

The composition of the present invention may further comprise from about 0.01% to about 5%, preferably from about 0.05% to about 2%, more preferably from about 0.07% to about 1% by weight of the total composition of an amphiphilic polymer selected from the groups consisting of amphiphilic alkoxylated polyalkyleneimine and mixtures thereof, preferably an amphiphilic alkoxylated polyalkyleneimine.

Preferably, the amphiphilic alkoxylated polyalkyleneimine is an alkoxylated polyethyleneimine polymer comprising a polyethyleneimine backbone having average molecular weight range from 100 to 5,000, preferably from 400 to 2,000, more preferably from 400 to 1,000 Daltons and the alkoxylated polyethyleneimine polymer further comprising:

(i) one or two alkoxylation modifications per nitrogen atom by a polyalkoxylene chain having an average of about 1 to about 50 alkoxy moieties per modification, wherein the terminal alkoxy moiety of the alkoxylation modification is capped with hydrogen, a C1-C4 alkyl or mixtures thereof;
(ii) an addition of one C1-C4 alkyl moiety and one or two alkoxylation modifications per nitrogen atom by a polyalkoxylene chain having an average of about 1 to about 50 alkoxy moieties per modification wherein the terminal alkoxy moiety is capped with hydrogen, a C1-C4 alkyl or mixtures thereof; or
(iii)a combination thereof; and

wherein the alkoxy moieties comprises ethoxy (EO) and/or propxy (PO) and/or butoxy (BO) and wherein when the alkoxylation modification comprises EO it also comprises PO or BO.

Preferred amphiphilic alkoxylated polyethyleneimine polymers comprise EO and PO groups within their alkoxylation chains, the PO groups preferably being in terminal position of the alkoxy chains, and the alkoxylation chains preferably being hydrogen capped. Hydrophilic alkoxylated polyethyleneimine polymers solely comprising ethoxy (EO) units within the alkoxylation chain could also optionally be formulated within the scope of this invention.

For example, but not limited to, below is shown possible modifications to terminal nitrogen atoms in the polyethyleneimine backbone where R represents an ethylene spacer and E represents a C1-C4 alkyl moiety and X- represents a suitable water soluble counterion.

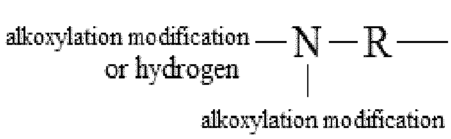

or

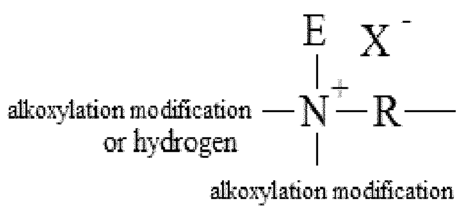

Also, for example, but not limited to, below is shown possible modifications to internal nitrogenatoms in the polyethyleneimine backbone where R represents an ethylene spacer and E represents a $C_1$-$C_4$ alkyl moiety and X- represents a suitable water soluble counterion.

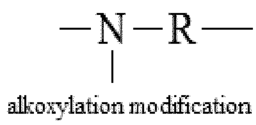

or

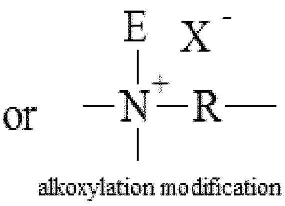

The alkoxylation modification of the polyethyleneimine backbone consists of the replacement of a hydrogen atom by a polyalkoxylene chain having an average of about 1 to about 50 alkoxy moieties, preferably from about 20 to about 45 alkoxy moieties, most preferably from about 30 to about 45 alkoxy moieties. The alkoxy moieties are selected from ethoxy (EO), propoxy (PO),butoxy (BO), and mixtures thereof. Alkoxy moieties solely comprising ethoxy units are outside the scope of the invention though. Preferably, the polyalkoxylene chain is selected from ethoxy/propoxy block moieties. More preferably, the polyalkoxylene chain is ethoxy/propoxy block moieties having an average degree of ethoxylation from about 3 to about 30 and an average degree of propoxylation from about 1 to about 20, more preferably ethoxy/propoxy block moieties having an average degree of ethoxylation from about 20 to about 30 and an average degree of propoxylation from about 10 to about 20.

More preferably the ethoxy/propoxy block moieties have a relative ethoxy to propoxy unit ratio between 3 to 1 and 1 to 1, preferably between 2 to 1 and 1 to 1. Most preferably the polyalkoxylene chain is the ethoxy/propoxy block moieties wherein the propoxy moiety block is the terminal alkoxy moiety block.

The modification may result in permanent quaternization of the polyethyleneimine backbone nitrogen atoms. The degree of permanent quaternization may be from 0% to about 30% of the polyethyleneimine backbone nitrogen atoms. It is preferred to have less than 30% of the polyethyleneimine backbone nitrogen atoms permanently quaternized. Most preferably the degree of quaternization is about 0%.

A preferred polyethyleneimine has the general structure of Formula (II):

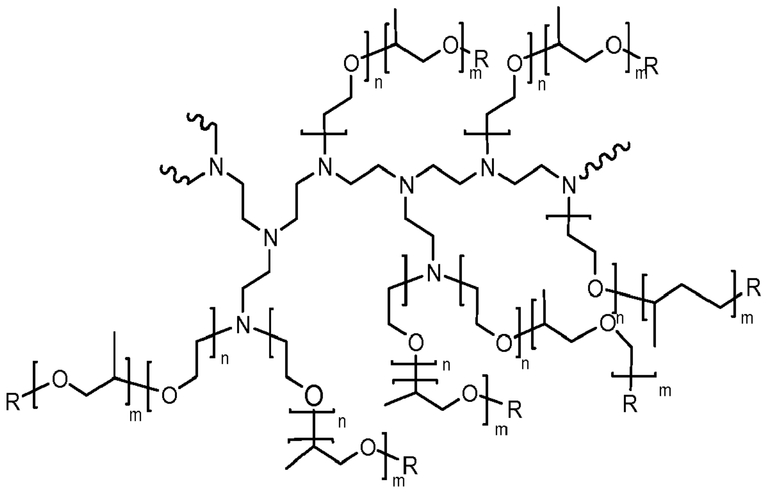

(II)

wherein the polyethyleneimine backbone has a weight average molecular weight of about 600, n of formula (II) has an average of about 10, m of formula (II) has an average of about 7 and R of formula (II) is selected from hydrogen, a $C_1$-$C_4$ alkyl and mixtures thereof, preferably hydrogen. The degree of permanent quaternization of formula (II) may be from 0% to about 22% of the polyethyleneimine backbone nitrogen atoms. The molecular weight of this polyethyleneimine preferably is between 10,000 and 15,000.

An alternative polyethyleneimine has the general structure of Formula (II) but wherein the polyethyleneimine backbone has a weight average molecular weight of about 600, n of Formula (II) has an average of about 24, m of Formula (II) has an average of about 16 and R of Formula (II) is selected from hydrogen, a $C_1$-$C_4$ alkyl and mixtures thereof, preferably hydrogen. The degree of permanent quaternization of Formula (II) may be from 0% to about 22% of the polyethyleneimine backbone nitrogen atoms. The molecular weight of this polyethyleneimine preferably is between 25,000 and 30,000.

Most preferred polyethyleneimine has the general structure of Formula (II) wherein the polyethyleneimine backbone has a weight average molecular weight of about 600, n of Formula (II) has an average of about 24, m of Formula (II) has an average of about 16 and R of Formula (II) is hydrogen. The degree of permanent quaternization of

Formula (II) is 0% of the polyethyleneimine backbone nitrogen atoms. The molecular weight of this polyethyleneimine preferably is about from about 25,000 to 30,000, most preferably about 28,000.
These polyethyleneimines can be prepared, for example, by polymerizing ethyleneimine in the presence of a catalyst such as carbon dioxide, sodium bisulfite, sulfuric acid, hydrogen peroxide, hydrochloric acid, acetic acid, and the like, as described in more detail in PCT Publication No. WO 2007/135645.

Chelant

The detergent composition herein can comprise a chelant at a level of from 0.1% to 20%, preferably from 0.2% to 5%, more preferably from 0.2% to 3% by weight of total composition.
As commonly understood in the detergent field, chelation herein means the binding or complexation of a bi- or multidentate ligand. These ligands, which are often organic compounds, are called chelants, chelators, chelating agents, and/or sequestering agent. Chelating agents form multiple bonds with a single metal ion. Chelants, are chemicals that form soluble, complex molecules with certain metal ions, inactivating the ions so that they cannot normally react with other elements or ions to produce precipitates or scale, or forming encrustations on soils turning them harder to be removed. The ligand forms a chelate complex with the substrate. The term is reserved for complexes in which the metal ion is bound to two or more atoms of the chelants.
Preferably, the composition of the present invention comprises one or more chelants, preferably selected from the group comprising carboxylate chelants, amino carboxylate chelants, amino phosphonate chelants, and mixtures thereof. Preferably the chelants are selected from the group consisting of MGDA (methylglycine-N,N-diacetic acid), GLDA (glutamic-N,N- diacetic acid), and mixtures thereof.
Suitable chelating agents can be selected from the group consisting of amino carboxylates, amino phosphonates, polycarboxylate chelating agents and mixtures thereof.
Other chelants include homopolymers and copolymers of polycarboxylic acids and their partially or completely neutralized salts, monomeric polycarboxylic acids and hydroxycarboxylic acids and their salts. Suitable polycarboxylic acids are acyclic, alicyclic, heterocyclic and aromatic carboxylic acids, in which case they contain at least two carboxyl groups which are in each case separated from one another by, preferably, no more than two carbon atoms. A suitable hydroxycarboxylic acid is, for example, citric acid. Another suitable polycarboxylic acid is the homopolymer of acrylic acid. Preferred are the polycarboxylates end capped with sulfonates.

Adjunct Ingredients

The detergent composition herein may optionally comprise a number of other adjunct ingredients such as builders (*e.g.*, preferably citrate), cleaning solvents, cleaning amines, conditioning polymers, cleaning polymers, surface modifying polymers, soil flocculating polymers, structurants, emollients, humectants, skin rejuvenating actives, enzymes, carboxylic acids, scrubbing particles, bleach and bleach activators, perfumes, malodor control agents, pigments, dyes, opacifiers, beads, pearlescent particles, microcapsules, inorganic cations such as alkaline earth metals such as Ca/Mg-ions, antibacterial agents, preservatives, viscosity adjusters (*e.g.*, salt such as NaCl, and other mono-, di- and trivalent salts) and pH adjusters and buffering means (*e.g.*, carboxylic acids such as citric acid, HCl, NaOH, KOH, alkanolamines, phosphoric and sulfonic acids, carbonates such as sodium carbonates, bicarbonates, sesquicarbonates, borates, silicates, phosphates, imidazole and alike).

Method of Washing

In another aspect, the invention is directed to a method of manually washing dishware comprising the steps of delivering a detergent composition of the invention into a volume of water to form a wash solution and immersing the dishware in the solution. As such, the composition herein will be applied in its diluted form to the dishware. Soiled surfaces *e.g.* dishes are contacted with an effective amount, typically from 0.5 mL to 20 mL (per 25 dishes being treated), preferably from 3mL to 10 mL, of the detergent composition of the present invention, preferably in liquid form, diluted in water. The actual amount of detergent composition used will be based on the judgment of user, and will typically depend upon factors such as the particular product formulation of the composition, including the concentration of active ingredients in the composition, the number of soiled dishes to be cleaned, the degree of soiling on the dishes, and the like. Generally, from 0.01 mL to 150 mL, preferably from 3 mL to 40 mL of a liquid detergent composition of the invention is combined with from 2,000 mL to 20,000 mL, more typically from 5,000 mL to 15,000 mL of water in a sink having a volumetric capacity in the range of from 1,000 mL to 20,000 mL, more typically from 5,000 mL to 15,000 mL. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish

surface for a period of time ranged from 1 to 10 seconds, although the actual time will vary with each application and user. The contacting of cloth, sponge, or similar article to the surface is preferably accompanied by a concurrent scrubbing of the surface.

In another aspect, the invention is directed to a method of manually washing dishware with the composition of the present invention. The method comprises the steps of: i) delivering a composition of the present invention onto the dishware or a cleaning implement; ii) cleaning the dishware with the composition in the presence of water; and iii) optionally, rinsing the dishware. The delivering step is preferably either directly onto the dishware surface or onto a cleaning implement, *i.e.*, in a neat form. The cleaning device or implement is preferably wet before or after the composition is delivered to it. Especially good grease removal has been found when the composition is used in neat form.

In another aspect, the invention is directed to a method of manually washing soiled articles comprising contacting a detergent composition of the invention with a surface, wherein the composition comprises a modified soy protein, and wherein the composition modifies the hydrophobicity of the surface as a result of the contacting step.

Another aspect of the present invention is directed to a method of promoting suds longevity or grease emulsification in a washing process for washing soiled articles, preferably dishware or fabric. The method comprises the steps of: a) delivering a detergent composition comprising a modified protein and a surfactant system to a volume of water to form a wash liquor; and b) immersing the soiled articles into said wash liquor. Preferably, the modified protein is present at a concentration of about 0.005 ppm to about 60 ppm, preferably at a concentration of about 0.02 ppm to about 12 ppm, in an aqueous wash liquor during the washing process.

Another aspect of the present invention is the use of a chemically or physically modified soy protein selected from the group consisting of (a) a chemically or physically modified soy 2S conglycinin, and (b) a chemically or physically modified soy 7S Beta-conglycinin, modified soy 11S glycinin, to provide increased suds longevity or increased grease emulsification in an aqueous wash liquor during a washing process. Preferably the chemically or physically modified soy 2S conglycinin is derived from an unmodified soy 2S conglycinin which has at least at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of a native soy 2S conglycinin protein of SEQ ID NO: 1. Preferably the chemically or physically modified soy 7S beta-conglycinin is derived from an unmodified soy 7S beta-conglycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of a native soy 7S beta-conglycinin selected from the group consisting of: SEQ ID NO: 2, SEQ ID NO: 3, and SEQ ID NO: 4.

Preferably the use of the chemically or physically modified soy protein wherein the chemically or physically modified soy protein comprises:

a) from 20 wt% to 80 wt%, preferably from 40 wt% to 60 wt%, by weight of the total chemically or physically modified soy protein, based on active protein, of a chemically or physically modified soy 2S conglycinin; and
b) from 20 wt% to 80 wt%, preferably from 40 wt% to 60 wt%, by weight of the total chemically or physically modified soy protein, based on active protein, of a chemically or physically modified soy 7S beta-conglycinin.

Preferably the use of the chemically or physically modified soy protein as described above further comprising a chemically or physically modified soy 11S glycinin, preferably as modified by the procedures as shown in Table 2 of the description. Preferably the chemically or physically modified soy 11S glycinin is derived from an unmodified soy 11S glycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of at least one reference sequence selected from a native soy 11S glycinin protein selected from the group consisting of SEQ ID NO: 5, and SEQ ID NO: 6.

Preferably the use of the chemically or physically modified soy protein as described above further comprising a chemically or physically modified soy 15S glycinin, preferably as modified by the procedures as shown in Table 2 of the description. Preferably, the chemically or physically modified soy 15S glycinin is derived from an unmodified soy 15S glycinin which is a dimer of the umodified soy 11S glycinin, wherein each dimer has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of at least one reference sequence selected from a native soy 11S glycinin protein selected from the group consisting of SEQ ID NO: 5, and SEQ ID NO: 6.

TEST METHODS

The following assay set forth must be used in order that the invention described and claimed herein may be

more fully understood.

Test Method 1: Suds Mileage

The evolution of the suds volume generated by a certain solution of dishwashing liquid is followed at specified 15 dh hardness, solution temperature at 46°C, and detergent concentrations (0.12 wt%), under influence of periodic soil injections. Data are compared and expressed versus a reference product as a suds mileage index (reference product has suds mileage index of 100).

A defined amount of dishwashing product depending on the targeted detergent concentration (0.12 wt%) is dispensed through a pipette with a flow rate of 0.67 mL/ sec at a height of 37 cm above the sink bottom surface into a water stream that starts filling up a sink (dimensions : cylinder - diameter 300 mm & height 288 mm) to 4 L with a constant pressure of 4 bar. With this pressure an initial suds volume is generated in the sink.

After recording the initial suds volume (average suds height * sink surface area) a fixed amount of greasy soil (6 mL) according to Table 1 will be injected instantaneously in the middle of the sink, while a paddle (metal blade 10 cm x 5 cm, positioned in the middle of the sink at the air liquid interface under an angle of 45 degrees) will rotate 20 times into the solution at 85 RPM. This step is followed immediately by another measurement of the total suds volume. The soil injecting, paddling and measuring steps are repeated until the measured suds volume reaches a minimum level, which is set at 400 $cm^3$. The amount of soil additions needed to get to that level is considered as the mileage of that specific sample.

The complete process is repeated 4 times per sample and per testing condition (temperature, concentration, hardness, soil type). As a final result the average mileage of the 4 replicates is calculated for each sample per soil type and averaged across testing conditions. Comparing the average mileage of the test sample versus that of the reference sample, indicates the performance of the test sample versus the reference sample, and is expressed as a suds mileage index, calculated as (average number of soil additions of test sample / average number of soil additions of reference sample) *100.

Table 1 - Greasy Soil Composition

| **Ingredient** | **Weight %** |
|---|---|
| Crisco oil | 12.730 |
| Crisco shortening | 27.752 |
| Lard | 7.638 |
| Refined Rendered Edible Beef Tallow | 51.684 |
| Oleic Acid, 90% (Techn) | 0.139 |
| Palmitic Acid, 99+% | 0.036 |
| Stearic Acid, 99+% | 0.021 |

EXAMPLES

The following examples are provided to further illustrate the present invention and are not to be construed as limitations of the present invention, as many variations of the present invention are possible without departing from its spirit or scope.

Example 1: Soy Protein Modifications

The soy proteins of the invention are modified separately or as a blend of soy proteins. The soy proteins are modified according to the processes described in Table 2, or a mixture thereof.

Table 2 - Modifications

| | | |
|---|---|---|
| 1 | Desugarization | Desugarization is achieved by treatment of food-grade baker's yeast (*Saccharomyces cerevisiae*) for 3 hrs at 30°C, and adjustment of the pH to 7 with citric or lactic acid. Desugarization can also occur by other bacterial treatment (*e.g.*, with *lactobacillus brevis*, *lactobacillus casei, streptococcus diacetilactis*, or *klebsiella Pneumoniae*) or by enzymatic treatment (*e.g.*, Glucose oxidase (EC 1.13.4)) with or without catalase (EC 1.11.1.6) and with or without $H_2O_2$. |
| 2 | Denaturation by heat | Dried soy proteins with remaining water content below 10% are submitted to a thermal treatment at 80°C for 96 hrs. |
| 3 | Denaturation by pH | Soy proteins are subjected to extreme pH to promote unfolding and expose hydrophobic residues to the protein surface. Preferred pH treatment is performed with IN NaOH to adjust the pH to 10-13, preferably 12 for at least 5 minutes at 20°C before formulating into the detergent composition. Alternatively acidic pH treatment is performed with 1N HCl to adjust the pH to 1-4, preferably 2 for at least 5 minutes at 20°C before formulating into the detergent composition. |
| 4 | Denaturation by sonication | A 10% soy protein solution is adjusted to pH 8 and sonicated with a VWR sonicator at 35 kHz for 3 hrs at 20°C. The pH of the sonicated soy protein solution is adjusted to pH 9 prior to usage. |
| 5 | Chemical Denaturation | A 10% soy protein solution is treated with 8M Urea and 1M $Na_2SO_3$ at 38°C for 6 hrs. Solution is dialyzed with 1% AES surfactant at pH 9 prior to usage. |
| 6 | Chemical modification | A 10% soy protein solution is esterified with 5% phenyl chloroformate at 20°C for 1 hr. The pH of the soy solution is adjusted continually during the reaction with IN NaOH to pH 4. At the end of the reaction, the pH is restored to pH 9, prior to usage. |
| 7 | Hydrolysis | A 10% soy protein solution is adjusted with IN NaOH to pH 7 and treated with 0.3% papain enzyme for 1hr at 35°C. Other suitable enzymes include ficin, trypsin, or bromelain. |

Example 2: Suds Mileage Performance

The ability to maintain suds mileage is assessed for detergent composition of the present invention (Inventive Ex. 1) comprising modified soy proteins according to Table 3. In parallel, comparative composition (Comparative Ex. 1) is made with unmodified soy proteins. The foregoing compositions are produced through standard mixing of the components described in Table 3.

Table 3 - Exemplary Detergent Compositions**

| **Ingredients** | **Inventive Ex.1** | **Comparative Ex. 1** |
|---|---|---|
| Sodium alkyl ethoxy sulfate (C1213EO0.6S) | 22.91% | 22.91% |
| n-C 12-14 Di Methyl Amine Oxide | 7.64% | 7.64% |
| Lutensol® XP80 (non-ionic surfactant supplied by BASF) | 0.45% | 0.45% |
| Sodium Chloride | 1.2% | 1.2% |
| Poly Propylene Glycol | 1% | 1% |
| Ethanol | 2% | 2% |
| Sodium Hydroxide | 0.24% | 0.24% |
| Modified 2S conglycinin* | 0.28% | - |
| Modified 7S beta-conglycinin* | 0.65% | - |
| Modified 11S glycinin* | 0.97% | - |
| Modified 15S glycinin* | 0.09% | - |
| Unmodified 2S conglycinin* | - | 0.28% |
| Unmodified 7S beta-conglycinin* | - | 0.65% |

(continued)

| Ingredients | Inventive Ex.1 | Comparative Ex. 1 |
|---|---|---|
| Unmodified 11S glycinin* | - | 0.97% |
| Unmodified 15S glycinin* | - | 0.09% |
| Minors (perfume, preservative, dye) + water | To 100% | To 100 % |
| pH (@ 0.12% solution) | 9 | 9 |
| *Note: proteins from soy protein isolate. | | |

The resultant compositions are assessed according to the Suds Mileage Index test method as described herein. The suds mileage results are summarized in Table 4. The higher the Suds Mileage Index value, the better in maintaining suds mileage. From the data it can be concluded that the Inventive Composition Ex. 1 comprising modified soy proteins according to the invention, wherein the modification is is desugarization as described in Table 2, has a stronger suds robustness in presence of greasy soils as compared to Comparative Composition Ex. 1 outside the scope of the invention (*i.e.*, comprising the native unmodified soy proteins).

Table 4 - Suds Mileage Index Results of Inventive and Comparative Compositions

| | Comparative Comp. Ex. 1 | Inventive Comp. Ex. 2 |
|---|---|---|
| Suds Mileage Index (Greasy Soil) | 100 | 121 |

Further, the suds mileage results are summarized in Table 5 for the Inventive Comp. Ex. 1a (with no $MgCl_2$), Inventive Comp. Ex. 1b (with 0.5% $MgCl_2$), and Inventive Comp. Ex. 1c (with 0.2% $MgCl_2$). Inventive Comp. Exs. 1a-1c are based off the Inventive Ex. 1 formulation contained in Table 3 and the modified soy proteins have been modified by desugarization as described in Table 2. From the data it can be concluded that single variable presence of multivalent metal cations ($Mg^{2+}$) further enhances the suds mileage benefit of the modified soy protein.

Table 5 - Suds Mileage Index Results of Inventive Compositions with $MgCl_2$

| | Inventive Comp. Ex. 1a | Inventive Comp. Ex. 1b | Inventive Comp. Ex. 1c |
|---|---|---|---|
| $MgCl_2$ | 0% | 0.05% | 0.2% |
| Suds Mileage Index (Greasy Soil) | 100 | 126 | 130 |

All percentages and ratios given for enzymes are based on active protein. All percentages and ratios herein are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

**Claims**

1. A detergent composition comprising:

    (i) a chemically or physically modified soy protein selected from the group consisting of:

        (a) from 20 wt% to 80 wt%, preferably from 30 wt% to 60 wt%, by weight of the chemically or physically modified soy protein, based on active protein, of a chemically or physically modified soy 2S conglycinin, preferably as modified by the procedures as shown in Table 2 of the description, preferably the chemically or physically modified soy 2S conglycinin is derived from an unmodified soy 2S conglycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of a native soy 2S conglycinin protein of SEQ ID NO: 1; and
        (b) from 20 wt% to 80 wt%, preferably from 40 wt% to 70 wt%, by weight of the chemically or physically modified soy protein, based on active protein, of a chemically or physically modified soy 7S beta-conglycinin, preferably as modified by the procedures as shown in Table 2 of the description, preferably the chemically or physically modified soy 7S beta-conglycinin is derived from an unmodified soy 7S beta-conglycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of at least one reference sequence selected from a native soy 7S beta-conglycinin selected from the group consisting of: SEQ ID NO: 2, SEQ ID NO: 3, and SEQ ID NO: 4; and
        wherein the sum total wt% of the chemically or physically modified soy 2S conglycinin, and the chemically or physically modified soy 7S beta-conglycinin add up to 100 wt% by weight of the total chemically or physically modified soy protein, based on active protein; and

    (ii) a surfactant system.

2. The composition according to claim 1, wherein the chemically or physically modified soy protein is a partially or completely denatured soy protein, preferably the denatured soy protein has been denatured by:

    i) application of heat to an unmodified soy protein at a temperature of from 60°C to 100°C;
    ii) pH treatment of an unmodified soy protein to a pH of from 1 to 4 or from 9 to 13;
    iii) chemical denaturation of an unmodified soy protein;
    iv) subjecting an unmodified soy protein to sonication, preferably high frequency sonication, in a range of from 5 kHz to 50 kHz; or
    v) combinations of i) to iv).

3. The composition according to any preceding claims, wherein the chemically or physically modified soy protein is derived from partial or complete desugarization of an unmodified soy protein, preferably the desugarization being performed with enzyme, bacteria or yeast, preferably by *S. cerevisiae.*

4. The composition according to any preceding claims, wherein the chemically or physically modified soy protein is derived from partial or complete esterification of an unmodified soy protein with C1-C12 linear, cyclic or aromatic alcohol, or with chloroformate, preferably the esterification being performed with chloroformate, preferably phenyl chloroformate optionally blended with phenol.

5. The composition according to any preceding claims, wherein the chemically or physically modified soy protein is a partially hydrolyzed soy protein, preferably derived from partial hydrolysis of an unmodified soy protein, preferably the partial hydrolysis being performed enzymatically by protease and the degree of hydrolyzation is 50% or less, preferably 20% or less, more preferably 10% or less, preferably the protease is papain.

6. The composition according to any preceding claims, wherein the chemically or physically modified soy protein has a weight average molecular weight of 2,000 kDa to 75,000 kDa.

7. The composition according to any preceding claims, wherein the chemically or physically modified soy 7S beta-conglycinin is a blend selected from the group consisting of a chemically or physically modified soy 7S beta-conglycinin alpha chain, a chemically or physically modified soy 7S beta-conglycinin alpha' chain, a chemically or physically modified soy 7S beta-conglycinin beta chain, and mixtures thereof.

8. The composition according to any preceding claims, wherein the chemically or physically modified soy protein is present in an amount of from 0.01 wt% to 5 wt%, preferably from 0.1% to 2.5 wt%, by weight of the composition, based on active protein.

9. The composition according to any preceding claims, wherein the composition has a pH of from 7 to 10, preferably from 8.5 to 9, when measured as a 10% product concentration in demineralized water at 20°C.

10. The composition according to any preceding claims, wherein the surfactant system is present in an amount of from 1 wt% to 60 wt%, preferably from 5 wt% to 50 wt%, more preferably from 8 wt% to 40 wt%, by weight of the composition, and wherein the surfactant system comprises one or more anionic surfactants and one or more co-surfactants and wherein the weight ratio of the anionic surfactants to the co-surfactants is less than 9:1, more preferably from 5:1 to 1:1, more preferably from 4:1 to 2:1, even more preferably from 3:1 to 2.5:1, and wherein the co-surfactants are selected from the group consisting of amphoteric surfactant, zwitterionic surfactant, and mixtures thereof, preferably the amphoteric surfactant is amine oxide surfactant and the zwitterionic surfactant is betaine surfactant.

11. The composition according to any preceding claims, further comprising a multivalent metal cation, preferably the multivalent metal cation is magnesium, aluminium, copper, calcium or iron, more preferably magnesium, most preferably the multivalent salt is magnesium chloride present in the amount of from 0.01 wt% to 2 wt% by weight of the composition.

12. A detergent composition comprising:

(i) a chemically or physically modified soy protein selected from the group consisting of:

(a) from 6 wt% to 56 wt%, preferably from 12 wt% to 36 wt%, by weight of the total chemically or physically modified soy protein, based on active protein, of a chemically or physically modified soy 2S conglycinin, preferably as modified by the procedures as shown in Table 2 of the description;
(b) from 6 wt% to 56 wt%, preferably from 16 wt% to 42 wt%, by weight of the total chemically or physically modified soy protein, based on active protein, of a chemically or physically modified soy 7S beta-conglycinin, preferably as modified by the procedures as shown in Table 2 of the description; and
(c) from 30 wt% to 70 wt%, preferably from 40 wt% to 60 wt%, by weight of the total chemically or physically modified soy protein, based on active protein, of a chemically or physically modified soy 11S glycinin, preferably as modified by the procedures as shown in Table 2 of the description;
wherein the sum total wt% of the chemically or physically modified soy 2S conglycinin, the chemically or physically modified soy 7S beta-conglycinin, and the chemically or physically modified soy 11S glycinin add up to 100 wt% by weight of the total chemically or physically modified soy protein, based on active protein; and

(ii) a surfactant system.

**13.** A detergent composition comprising:

(i) a chemically or physically modified soy protein selected from the group consisting of:

(a) from 5 wt% to 55 wt%, preferably from 11 wt% to 35 wt% by weight of the total chemically or physically modified soy protein, based on active protein, of a chemically or physically modified soy 2S conglycinin, preferably as modified by the procedures as shown in Table 2 of the description;
(b) from 5 wt% to 55 wt%, preferably from 15 wt% to 41 wt%, by weight of the total chemically or physically modified soy protein, based on active protein, of a chemically or physically modified soy 7S beta-conglycinin, preferably as modified by the procedures as shown in Table 2 of the description;
(c) from 24 wt% to 69 wt%, preferably from 36 wt% to 59 wt% by weight of the total chemically or physically modified soy protein, based on active protein, of a chemically or physically modified soy 11S glycinin, preferably as modified by the procedures as shown in Table 2 of the description; and
(d) from 1 wt% to 20 wt%, preferably from 1 wt% to 10 wt%, by weight of the total chemically or physically modified soy protein, based on active protein, of a chemically or physically modified soy 15S glycinin, preferably as modified by the procedures as shown in Table 2 of the description;
wherein the sum total wt% of the chemically or physically modified soy 2S conglycinin, the chemically or physically modified soy 7S beta-conglycinin, the chemically or physically modified soy 11S glycinin, and the chemically or physically modified soy 15S glycinin add up to 100 wt%, by weight of the total chemically or physically modified soy protein, based on active protein; and

(ii) a surfactant system.

**14.** A method of manually washing dishware comprising the steps of delivering a detergent composition according to any preceding claims into a volume of water to form a wash solution and immersing the dishware in the solution.

**15.** Use of a chemically or physically modified soy protein selected from the group consisting of: (a) a chemically or physically modified soy 2S conglycinin, preferably as modified by the procedures as shown in Table 2 of the description, preferably the modified soy 2S conglycinin is derived from an unmodified soy 2S conglycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of a native soy 2S conglycinin protein of SEQ ID NO: 1; and (b) a chemically or physically modified soy 7S beta-conglycinin, preferably as modified by the procedures as shown in Table 2 of the description, preferably the modified soy 7S beta-conglycinin is derived from an unmodified soy 7S beta-conglycinin which has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of a sequence aligned against the entire length of at least one reference sequence selected from a native soy 7S beta-conglycinin selected from the group consisting of: SEQ ID NO: 2, SEQ ID NO: 3, and SEQ ID NO: 4, to provide increased suds longevity or increased grease emulsification in an aqueous wash liquor during a washing process.

Europäisches Patentamt
European Patent Office
Office européen des brevets

# EUROPEAN SEARCH REPORT

Application Number
EP 18 18 0838

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GB 1 551 074 A (PROCTER & GAMBLE) 22 August 1979 (1979-08-22) * page 1, lines 6-11 * * page 1, line 33 - page 2, line 4 * * page 3, line 61 - page 5, line 4 * * page 5, line 61 - page 7222 * * page 10; claims; example VI; table VI * ----- | 1-15 | INV. C11D1/94 C07K14/415 C11D3/38 C11D11/00 |
| Y | GB 1 529 841 A (PROCTER & GAMBLE LTD) 25 October 1978 (1978-10-25) * examples V, X * ----- | 1-15 | |
| Y | Miroljub Barac ET AL: "Soy protein modification: A review", Acta periodica technologica, 1 January 2004 (2004-01-01), pages 3-16, XP055470839, DOI: 10.2298/APT0435003B Retrieved from the Internet: URL:https://core.ac.uk/download/pdf/27170482.pdf * page 4, paragraph 2 - page 5, paragraph 1 * * page 6, paragraph 2 - page 11, paragraph 2 * ----- -/-- | 1-15 | TECHNICAL FIELDS SEARCHED (IPC): C11D C07K |

The present search report has been drawn up for all claims

| Place of search | Date of completion of the search | Examiner |
|---|---|---|
| The Hague | 11 February 2019 | Marttin, Emmeline |

3

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 3

Europäisches Patentamt
European Patent Office
Office européen des brevets

# EUROPEAN SEARCH REPORT

Application Number
EP 18 18 0838

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | K. NISHINARI ET AL: "Soy proteins: A review on composition, aggregation and emulsification", FOOD HYDROCOLLOIDS, vol. 39, 1 August 2014 (2014-08-01), pages 301-318, XP055470832, NL ISSN: 0268-005X, DOI: 10.1016/j.foodhyd.2014.01.013 * page 302, left-hand column, paragraph 5 * * page 314, left-hand column, last paragraph - right-hand column, paragraph 1 * ----- | 1-15 | |
| Y | MIRYAM AMIGO-BENAVENT ET AL: "Digestibility and immunoreactivity of soybean -conglycinin and its deglycosylated form", FOOD CHEMISTRY, ELSEVIER LTD, NL, vol. 129, no. 4, 10 June 2011 (2011-06-10), pages 1598-1605, XP028262743, ISSN: 0308-8146, DOI: 10.1016/J.FOODCHEM.2011.06.015 [retrieved on 2011-06-17] * page 1599 - paragraph 2.2.1 * ----- | 1-15 | TECHNICAL FIELDS SEARCHED (IPC) |

-/--

The present search report has been drawn up for all claims

3

| Place of search | Date of completion of the search | Examiner |
|---|---|---|
| The Hague | 11 February 2019 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 18 18 0838

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | SHENGDI HU ET AL: "Development of Immunoaffinity Chromatographic Method for Isolating Glycinin (11S) from Soybean Proteins", JOURNAL OF AGRICULTURAL AND FOOD CHEMISTRY, vol. 61, no. 18, 26 April 2013 (2013-04-26), pages 4406-4410, XP055553926, US ISSN: 0021-8561, DOI: 10.1021/jf400009g * page 4406, left-hand column, paragraph 2-3 * * page 4408, left-hand column, paragraph 3 - page 4409, right-hand column, last paragraph * ----- | 1-15 | |
| Y | US 8 142 832 B2 (IOWA STATE UNIVERSITY RESEARCH FOUNDATION INC.) 27 March 2012 (2012-03-27) * column 1, last paragraph - column 3, paragraph 3 * ----- | 13 | TECHNICAL FIELDS SEARCHED (IPC) |
| Y | GERMAN ET AL: "Film forming and foaming behavior of food proteins", JOURNAL OF THE AMERICAN OIL CHEMISTS' SOCIETY (JAOCS), SPRINGER, DE , vol. 62, no. 9 1 September 1985 (1985-09-01), page 1358, XP009511109, ISSN: 0003-021X, DOI: 10.1007/BF02545958 Retrieved from the Internet: URL:https://link.springer.com/article/10.1007/BF02545958 * page 1363, left-hand column, last paragraph - right-hand column, last paragraph * ----- | 1-15 | |

The present search report has been drawn up for all claims

3

| Place of search | Date of completion of the search | Examiner |
|---|---|---|
| The Hague | 11 February 2019 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

Europäisches Patentamt
European Patent Office
Office européen des brevets

Application Number
EP 18 18 0838

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 18 18 0838

The Search Division considers that the present European patent application does not comply with the requirements of unity of invention and relates to several inventions or groups of inventions, namely:

1. claims: 1-15

A detergent composition comprising (i) a chemically or physically modified soy protein and (ii) a surfactant system. A method of manually washing dishware comprising the steps of delivering said detergent composition according to any preceding claims into a volume of water to form a wash solution and immersing the dishware in the solution. Use of a chemically or physically modified soy protein to provide increased suds longevity or increased grease emulsification in an aqueous wash liquor during a washing process.

1.1. claims: 1-11, 15(completely); 14(partially)

A detergent composition comprising: (i) a chemically or physically modified soy protein selected from the group consisting of: (a) from 20 wt% to 80 wt% by weight of the chemically or physically modified soy protein, based on active protein, of a chemically or physically modified soy 2S conglycinin; (b) 20 wt% to 80 wt% of a chemically or physically modified soy 7S beta-conglycinin ;and wherein the sum total wt% of the chemically or physically modified soy 2S conglycinin, and the chemically or physically modified soy 7S beta-conglycinin add up to 100 wt% by weight of the total chemically or physically modified soy protein, based on active protein; and (ii) a surfactant system. A method of manually washing dishware comprising the steps of delivering said detergent composition according to any preceding claims into a volume of water to form a wash solution and immersing the dishware in the solution. Use of a chemically or physically modified soy protein selected from the group (a) a chemically or physically modified soy 2S conglycinin and (b) a chemically or physically modified soy 7S beta-conglycinin, to provide increased suds longevity or increased grease emulsification in an aqueous wash liquor during a washing process.

1.2. claims: 12(completely); 14(partially)

A detergent composition comprising: (i) a chemically or physically modified soy protein selected from the group consisting of: (a) from 6 wt% to 56 wt% by weight of the total chemically or physically modified soy protein, based on active protein, of a chemically or physically modified soy 2S conglycinin; (b) from 6 wt% to 56 wt% by weight of the total chemically or physically modified soy protein, based on active protein, of a chemically or physically modified soy 7S beta-conglycinin; and (c) from 30 wt% to 70 wt% by weight of the total chemically or physically modified soy protein, based on active protein, of a chemically or physically modified soy 11 S glycinin, wherein the sum total

page 1 of 2

Europäisches Patentamt
European Patent Office
Office européen des brevets

# LACK OF UNITY OF INVENTION SHEET B

Application Number

EP 18 18 0838

The Search Division considers that the present European patent application does not comply with the requirements of unity of invention and relates to several inventions or groups of inventions, namely:

wt% of the chemically or physically modified soy 2S conglycinin, the chemically or physically modified soy 7S beta-conglycinin, and the chemically or physically modified soy 1 IS glycinin add up to 100 wt% by weight of the total chemically or physically modified soy protein, based on active protein; and (ii) a surfactant system. A method of manually washing dishware comprising the steps of delivering said detergent composition according to any preceding claims into a volume of water to form a wash solution and immersing the dishware in the solution.

1.3. claims: 13(completely); 14(partially)

A detergent composition comprising: (i) a chemically or physically modified soy protein selected from the group consisting of: (a) from 5 wt% to 55 wt% by weight of the total chemically or physically modified soy protein, based on active protein, of a chemically or physically modified soy 2S conglycinin; (b) from 5 wt% to 55 wt% by weight of the total chemically or physically modified soy protein, based on active protein, of a chemically or physically modified soy 7S beta-conglycinin; (c) from 24 wt% to 69 wt% by weight of the total chemically or physically modified soy protein, based on active protein, of a chemically or physically modified soy 11 S glycinin; and (d) from 1 wt% to 20 wt% by weight of the total chemically or physically modified soy protein, based on active protein, of a chemically or physically modified soy 15S glycinin; wherein the sum total wt% of the chemically or physically modified soy 2S conglycinin, the chemically or physically modified soy 7S beta-conglycinin, the chemically or physically modified soy 11 S glycinin, and the chemically or physically modified soy 15S glycinin add up to 100 wt%, by weight of the total chemically or physically modified soy protein, based on active protein; and (ii) a surfactant system. A method of manually washing dishware comprising the steps of delivering said detergent composition according to any preceding claims into a volume of water to form a wash solution and immersing the dishware in the solution.

---

Please note that all inventions mentioned under item 1, although not necessarily linked by a common inventive concept, could be searched without effort justifying an additional fee.

# ANNEX TO THE EUROPEAN SEARCH REPORT ON EUROPEAN PATENT APPLICATION NO. EP 18 18 0838

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2019

| Patent document cited in search report | Publication date | Patent family member(s) | Publication date |
|---|---|---|---|
| GB 1551074 A | 22-08-1979 | DE 2636967 A1 | 03-03-1977 |
| | | FR 2321538 A1 | 18-03-1977 |
| | | GB 1551074 A | 22-08-1979 |
| | | IT 1064960 B | 25-02-1985 |
| | | JP S5252908 A | 28-04-1977 |
| | | NL 7609221 A | 22-02-1977 |
| GB 1529841 A | 25-10-1978 | CA 1059002 A | 24-07-1979 |
| | | GB 1529841 A | 25-10-1978 |
| | | JP S51125406 A | 01-11-1976 |
| | | PH 13399 A | 28-03-1980 |
| US 8142832 B2 | 27-03-2012 | US 2008095914 A1 | 24-04-2008 |
| | | US 2012095190 A1 | 19-04-2012 |
| | | WO 2006047308 A2 | 04-05-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014018929 A
- WO 201219844 A
- WO 201219849 A
- WO 201219848 A
- US 3915903 A
- WO 2007135645 A


### Non-patent literature cited in the description


- **WOLF et al.** *J. Agric. Good. Chem.,* 1996, vol. 44, 785-791
- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919
- **PETERSEN TN ; BRUNAK S. ; VON HEIJNE G. ; NIELSEN H.** *Nature Methods,* 2011, vol. 8, 785-786
- **ROBERT LAUGHLIN.** The Aqueous Phase Behaviour of Surfactants. Academic Press, 1994, 538-542

SEQUENCE LISTING

<110> The Procter & Gamble Company

<120> Detergent composition

<130> CM04900FM

<150> EP17201326.0
<151> 2017-11-13

<160> 7

<170> PatentIn version 3.5

<210> 1
<211> 158
<212> PRT
<213> 2S conglycin (18.4kDa)

<400> 1

```
Met Thr Lys Phe Thr Ile Leu Leu Ile Ser Leu Leu Phe Cys Ile Ala
1               5                   10                  15

His Thr Cys Ser Ala Ser Lys Trp Gln His Gln Gln Asp Ser Cys Arg
            20                  25                  30

Lys Gln Leu Gln Gly Val Asn Leu Thr Pro Cys Glu Lys His Ile Met
        35                  40                  45

Glu Lys Ile Gln Gly Arg Gly Asp Asp Asp Asp Asp Asp Asp Asp Asp
    50                  55                  60

Asn His Ile Leu Arg Thr Met Arg Gly Arg Ile Asn Tyr Ile Arg Arg
65                  70                  75                  80

Asn Glu Gly Lys Asp Glu Asp Glu Glu Glu Glu Gly His Met Gln Lys
                85                  90                  95

Cys Cys Thr Glu Met Ser Glu Leu Arg Ser Pro Lys Cys Gln Cys Lys
            100                 105                 110

Ala Leu Gln Lys Ile Met Glu Asn Gln Ser Glu Glu Leu Glu Glu Lys
        115                 120                 125

Gln Lys Lys Lys Met Glu Lys Glu Leu Ile Asn Leu Ala Thr Met Cys
    130                 135                 140

Arg Phe Gly Pro Met Ile Gln Cys Asp Leu Ser Ser Asp Asp
145                 150                 155
```

<210> 2

<211> 639
<212> PRT
<213> Beta-conglycinin, alpha' chain (74.3Kda)

<400> 2

```
Met Met Arg Ala Arg Phe Pro Leu Leu Leu Leu Gly Val Val Phe Leu
1               5                   10                  15

Ala Ser Val Ser Val Ser Phe Gly Ile Ala Tyr Trp Glu Lys Gln Asn
            20                  25                  30

Pro Ser His Asn Lys Cys Leu Arg Ser Cys Asn Ser Glu Lys Asp Ser
        35                  40                  45

Tyr Arg Asn Gln Ala Cys His Ala Arg Cys Asn Leu Leu Lys Val Glu
    50                  55                  60

Glu Glu Glu Glu Cys Glu Glu Gly Gln Ile Pro Arg Pro Arg Pro Gln
65                  70                  75                  80

His Pro Glu Arg Glu Arg Gln Gln His Gly Glu Lys Glu Glu Asp Glu
                85                  90                  95

Gly Glu Gln Pro Arg Pro Phe Pro Phe Pro Arg Pro Arg Gln Pro His
            100                 105                 110

Gln Glu Glu Glu His Glu Gln Lys Glu Glu His Glu Trp His Arg Lys
        115                 120                 125

Glu Glu Lys His Gly Gly Lys Gly Ser Glu Glu Glu Gln Asp Glu Arg
    130                 135                 140

Glu His Pro Arg Pro His Gln Pro His Gln Lys Glu Glu Glu Lys His
145                 150                 155                 160

Glu Trp Gln His Lys Gln Glu Lys His Gln Gly Lys Glu Ser Glu Glu
                165                 170                 175

Glu Glu Glu Asp Gln Asp Glu Asp Glu Glu Gln Asp Lys Glu Ser Gln
            180                 185                 190

Glu Ser Glu Gly Ser Glu Ser Gln Arg Glu Pro Arg Arg His Lys Asn
        195                 200                 205

Lys Asn Pro Phe His Phe Asn Ser Lys Arg Phe Gln Thr Leu Phe Lys
    210                 215                 220

Asn Gln Tyr Gly His Val Arg Val Leu Gln Arg Phe Asn Lys Arg Ser
```

```
225                 230                 235                 240

Gln Gln Leu Gln Asn Leu Arg Asp Tyr Arg Ile Leu Glu Phe Asn Ser
                245                 250                 255

Lys Pro Asn Thr Leu Leu Leu Pro His His Ala Asp Ala Asp Tyr Leu
            260                 265                 270

Ile Val Ile Leu Asn Gly Thr Ala Ile Leu Thr Leu Val Asn Asn Asp
        275                 280                 285

Asp Arg Asp Ser Tyr Asn Leu Gln Ser Gly Asp Ala Leu Arg Val Pro
    290                 295                 300

Ala Gly Thr Thr Phe Tyr Val Val Asn Pro Asp Asn Asp Glu Asn Leu
305                 310                 315                 320

Arg Met Ile Ala Gly Thr Thr Phe Tyr Val Val Asn Pro Asp Asn Asp
                325                 330                 335

Glu Asn Leu Arg Met Ile Thr Leu Ala Ile Pro Val Asn Lys Pro Gly
            340                 345                 350

Arg Phe Glu Ser Phe Phe Leu Ser Ser Thr Gln Ala Gln Gln Ser Tyr
        355                 360                 365

Leu Gln Gly Phe Ser Lys Asn Ile Leu Glu Ala Ser Tyr Asp Thr Lys
    370                 375                 380

Phe Glu Glu Ile Asn Lys Val Leu Phe Gly Arg Glu Glu Gly Gln Gln
385                 390                 395                 400

Gln Gly Glu Glu Arg Leu Gln Glu Ser Val Ile Val Glu Ile Ser Lys
                405                 410                 415

Lys Gln Ile Arg Glu Leu Ser Lys His Ala Lys Ser Ser Ser Arg Lys
            420                 425                 430

Thr Ile Ser Ser Glu Asp Lys Pro Phe Asn Leu Gly Ser Arg Asp Pro
        435                 440                 445

Ile Tyr Ser Asn Lys Leu Gly Lys Leu Phe Glu Ile Thr Gln Arg Asn
    450                 455                 460

Pro Gln Leu Arg Asp Leu Asp Val Phe Leu Ser Val Val Asp Met Asn
465                 470                 475                 480
```

Glu Gly Ala Leu Phe Leu Pro His Phe Asn Ser Lys Ala Ile Val Val
485 490 495

Leu Val Ile Asn Glu Gly Glu Ala Asn Ile Glu Leu Val Gly Ile Lys
500 505 510

Glu Gln Gln Gln Arg Gln Gln Gln Glu Glu Gln Pro Leu Glu Val Arg
515 520 525

Lys Tyr Arg Ala Glu Leu Ser Glu Gln Asp Ile Phe Val Ile Pro Ala
530 535 540

Gly Tyr Pro Val Met Val Asn Ala Thr Ser Asp Leu Asn Phe Phe Ala
545 550 555 560

Phe Gly Ile Asn Ala Glu Asn Asn Gln Arg Asn Phe Leu Ala Gly Ser
565 570 575

Lys Asp Asn Val Ile Ser Gln Ile Pro Ser Gln Val Gln Glu Leu Ala
580 585 590

Phe Pro Arg Ser Ala Lys Asp Ile Glu Asn Leu Ile Lys Ser Gln Ser
595 600 605

Glu Ser Tyr Phe Val Asp Ala Gln Pro Gln Gln Lys Glu Glu Gly Asn
610 615 620

Lys Gly Arg Lys Gly Pro Leu Ser Ser Ile Leu Arg Ala Phe Tyr
625 630 635

<210> 3
<211> 439
<212> PRT
<213> Beta-conglycinin, beta chain (50.5Kda)

<400> 3

Met Met Arg Val Arg Phe Pro Leu Leu Val Leu Leu Gly Thr Val Phe
1 5 10 15

Leu Ala Ser Val Cys Val Ser Leu Lys Val Arg Glu Asp Glu Asn Asn
20 25 30

Pro Phe Tyr Phe Arg Ser Ser Asn Ser Phe Gln Thr Leu Phe Glu Asn
35 40 45

Gln Asn Val Arg Ile Arg Leu Leu Gln Arg Phe Asn Lys Arg Ser Pro
50 55 60

```
Gln Leu Glu Asn Leu Arg Asp Tyr Arg Ile Val Gln Phe Gln Ser Lys
65                  70                  75                  80

Pro Asn Thr Ile Leu Leu Pro His His Ala Asp Ala Asp Phe Leu Leu
                85                  90                  95

Phe Val Leu Ser Gly Arg Ala Ile Leu Thr Leu Val Asn Asn Asp Asp
            100                 105                 110

Arg Asp Ser Tyr Asn Leu His Pro Gly Asp Ala Gln Arg Ile Pro Ala
        115                 120                 125

Gly Thr Thr Tyr Tyr Leu Val Asn Pro His Asp His Gln Asn Leu Lys
    130                 135                 140

Ile Ile Lys Leu Ala Ile Pro Val Asn Lys Pro Gly Arg Tyr Asp Asp
145                 150                 155                 160

Phe Phe Leu Ser Ser Thr Gln Ala Gln Gln Ser Tyr Leu Gln Gly Phe
                165                 170                 175

Ser His Asn Ile Leu Glu Thr Ser Phe His Ser Glu Phe Glu Glu Ile
            180                 185                 190

Asn Arg Val Leu Phe Gly Glu Glu Glu Glu Gln Arg Gln Gln Glu Gly
        195                 200                 205

Val Ile Val Glu Leu Ser Lys Glu Gln Ile Arg Gln Leu Ser Arg Arg
    210                 215                 220

Ala Lys Ser Ser Ser Arg Lys Thr Ile Ser Ser Glu Asp Glu Pro Phe
225                 230                 235                 240

Asn Leu Arg Ser Arg Asn Pro Ile Tyr Ser Asn Asn Phe Gly Lys Phe
                245                 250                 255

Phe Glu Ile Thr Pro Glu Lys Asn Pro Gln Leu Arg Asp Leu Asp Ile
            260                 265                 270

Phe Leu Ser Ser Val Asp Ile Asn Glu Gly Ala Leu Leu Leu Pro His
        275                 280                 285

Phe Asn Ser Lys Ala Ile Val Ile Leu Val Ile Asn Glu Gly Asp Ala
    290                 295                 300

Asn Ile Glu Leu Val Gly Ile Lys Glu Gln Gln Gln Lys Gln Lys Gln
305                 310                 315                 320
```

```
Glu Glu Glu Pro Leu Glu Val Gln Arg Tyr Arg Ala Glu Leu Ser Glu
                325                 330                 335

Asp Asp Val Phe Val Ile Pro Ala Ala Tyr Pro Phe Val Val Asn Ala
            340                 345                 350

Thr Ser Asn Leu Asn Phe Leu Ala Phe Gly Ile Asn Ala Glu Asn Asn
        355                 360                 365

Gln Arg Asn Phe Leu Ala Gly Glu Lys Asp Asn Val Val Arg Gln Ile
    370                 375                 380

Glu Arg Gln Val Gln Glu Leu Ala Phe Pro Gly Ser Ala Gln Asp Val
385                 390                 395                 400

Glu Arg Leu Leu Lys Lys Gln Arg Glu Ser Tyr Phe Val Asp Ala Gln
                405                 410                 415

Pro Gln Gln Lys Glu Glu Gly Ser Lys Gly Arg Lys Gly Pro Phe Pro
            420                 425                 430

Ser Ile Leu Gly Ala Leu Tyr
        435


<210>  4
<211>  605
<212>  PRT
<213>  Beta-conglycinin, alpha chain (70.7 Kda)

<400>  4

Met Met Arg Ala Arg Phe Pro Leu Leu Leu Leu Gly Leu Val Phe Leu
1               5                   10                  15

Ala Ser Val Ser Val Ser Phe Gly Ile Ala Tyr Trp Glu Lys Glu Asn
            20                  25                  30

Pro Lys His Asn Lys Cys Leu Gln Ser Cys Asn Ser Glu Arg Asp Ser
        35                  40                  45

Tyr Arg Asn Gln Ala Cys His Ala Arg Cys Asn Leu Leu Lys Val Glu
    50                  55                  60

Lys Glu Glu Cys Glu Glu Gly Glu Ile Pro Arg Pro Arg Pro Arg Pro
65                  70                  75                  80

Gln His Pro Glu Arg Glu Pro Gln Gln Pro Gly Glu Lys Glu Glu Asp
                85                  90                  95
```

```
Glu Asp Glu Gln Pro Arg Pro Ile Pro Phe Pro Arg Pro Gln Pro Arg
            100                 105                 110

Gln Glu Glu Glu His Glu Gln Arg Glu Glu Gln Glu Trp Pro Arg Lys
        115                 120                 125

Glu Glu Lys Arg Gly Glu Lys Gly Ser Glu Glu Glu Asp Glu Asp Glu
    130                 135                 140

Asp Glu Glu Gln Asp Glu Arg Gln Phe Pro Phe Pro Arg Pro Pro His
145                 150                 155                 160

Gln Lys Glu Glu Arg Asn Glu Glu Glu Asp Glu Asp Glu Glu Gln Gln
                165                 170                 175

Arg Glu Ser Glu Glu Ser Glu Asp Ser Glu Leu Arg Arg His Lys Asn
            180                 185                 190

Lys Asn Pro Phe Leu Phe Gly Ser Asn Arg Phe Glu Thr Leu Phe Lys
        195                 200                 205

Asn Gln Tyr Gly Arg Ile Arg Val Leu Gln Arg Phe Asn Gln Arg Ser
    210                 215                 220

Pro Gln Leu Gln Asn Leu Arg Asp Tyr Arg Ile Leu Glu Phe Asn Ser
225                 230                 235                 240

Lys Pro Asn Thr Leu Leu Leu Pro Asn His Ala Asp Ala Asp Tyr Leu
                245                 250                 255

Ile Val Ile Leu Asn Gly Thr Ala Ile Leu Ser Leu Val Asn Asn Asp
            260                 265                 270

Asp Arg Asp Ser Tyr Arg Leu Gln Ser Gly Asp Ala Leu Arg Val Pro
        275                 280                 285

Ser Gly Thr Thr Tyr Tyr Val Val Asn Pro Asp Asn Asn Glu Asn Leu
    290                 295                 300

Arg Leu Ile Thr Leu Ala Ile Pro Val Asn Lys Pro Gly Arg Phe Glu
305                 310                 315                 320

Ser Phe Phe Leu Ser Ser Thr Glu Ala Gln Gln Ser Tyr Leu Gln Gly
                325                 330                 335

Phe Ser Arg Asn Ile Leu Glu Ala Ser Tyr Asp Thr Lys Phe Glu Glu
            340                 345                 350
```

Ile Asn Lys Val Leu Phe Ser Arg Glu Glu Gly Gln Gln Gln Gly Glu
355 360 365

Gln Arg Leu Gln Glu Ser Val Ile Val Glu Ile Ser Lys Glu Gln Ile
370 375 380

Arg Ala Leu Ser Lys Arg Ala Lys Ser Ser Ser Arg Lys Thr Ile Ser
385 390 395 400

Ser Glu Asp Lys Pro Phe Asn Leu Arg Ser Arg Asp Pro Ile Tyr Ser
405 410 415

Asn Lys Leu Gly Lys Phe Phe Glu Ile Thr Pro Glu Lys Asn Pro Gln
420 425 430

Leu Arg Asp Leu Asp Ile Phe Leu Ser Ile Val Asp Met Asn Glu Gly
435 440 445

Ala Leu Leu Leu Pro His Phe Asn Ser Lys Ala Ile Val Ile Leu Val
450 455 460

Ile Asn Glu Gly Asp Ala Asn Ile Glu Leu Val Gly Leu Lys Glu Gln
465 470 475 480

Gln Gln Glu Gln Gln Gln Glu Glu Gln Pro Leu Glu Val Arg Lys Tyr
485 490 495

Arg Ala Glu Leu Ser Glu Gln Asp Ile Phe Val Ile Pro Ala Gly Tyr
500 505 510

Pro Val Val Val Asn Ala Thr Ser Asn Leu Asn Phe Phe Ala Ile Gly
515 520 525

Ile Asn Ala Glu Asn Asn Gln Arg Asn Phe Leu Ala Gly Ser Gln Asp
530 535 540

Asn Val Ile Ser Gln Ile Pro Ser Gln Val Gln Glu Leu Ala Phe Pro
545 550 555 560

Gly Ser Ala Gln Ala Val Glu Lys Leu Leu Lys Asn Gln Arg Glu Ser
565 570 575

Tyr Phe Val Asp Ala Gln Pro Lys Lys Lys Glu Glu Gly Asn Lys Gly
580 585 590

Arg Lys Gly Pro Leu Ser Ser Ile Leu Arg Ala Phe Tyr

595 600 605

<210> 5
<211> 516
<212> PRT
<213> Glycinin (57.9 Kda)

<400> 5

Met Gly Lys Pro Phe Phe Thr Leu Ser Leu Ser Ser Leu Cys Leu Leu
1 5 10 15

Leu Leu Ser Ser Ala Cys Phe Ala Ile Thr Ser Ser Lys Phe Asn Glu
20 25 30

Cys Gln Leu Asn Asn Leu Asn Ala Leu Glu Pro Asp His Arg Val Glu
35 40 45

Ser Glu Gly Gly Leu Ile Glu Thr Trp Asn Ser Gln His Pro Glu Leu
50 55 60

Gln Cys Ala Gly Val Thr Val Ser Lys Arg Thr Leu Asn Arg Asn Gly
65 70 75 80

Ser His Leu Pro Ser Tyr Leu Pro Tyr Pro Gln Met Ile Ile Val Val
85 90 95

Gln Gly Lys Gly Ala Ile Gly Phe Ala Phe Pro Gly Cys Pro Glu Thr
100 105 110

Phe Glu Lys Pro Gln Gln Gln Ser Ser Arg Arg Gly Ser Arg Ser Gln
115 120 125

Gln Gln Leu Gln Asp Ser His Gln Lys Ile Arg His Phe Asn Glu Gly
130 135 140

Asp Val Leu Val Ile Pro Leu Gly Val Pro Tyr Trp Thr Tyr Asn Thr
145 150 155 160

Gly Asp Glu Pro Val Val Ala Ile Ser Pro Leu Asp Thr Ser Asn Phe
165 170 175

Asn Asn Gln Leu Asp Gln Asn Pro Arg Val Phe Tyr Leu Ala Gly Asn
180 185 190

Pro Asp Ile Glu His Pro Glu Thr Met Gln Gln Gln Gln Gln Gln Lys
195 200 205

Ser His Gly Gly Arg Lys Gln Gly Gln His Arg Gln Gln Glu Glu Glu

210 215 220

Gly Gly Ser Val Leu Ser Gly Phe Ser Lys His Phe Leu Ala Gln Ser
225 230 235 240

Phe Asn Thr Asn Glu Asp Thr Ala Glu Lys Leu Arg Ser Pro Asp Asp
245 250 255

Glu Arg Lys Gln Ile Val Thr Val Glu Gly Gly Leu Ser Val Ile Ser
260 265 270

Pro Lys Trp Gln Glu Gln Glu Asp Glu Asp Glu Asp Glu Asp Glu Glu
275 280 285

Tyr Gly Arg Thr Pro Ser Tyr Pro Pro Arg Arg Pro Ser His Gly Lys
290 295 300

His Glu Asp Asp Glu Asp Glu Asp Glu Glu Glu Asp Gln Pro Arg Pro
305 310 315 320

Asp His Pro Pro Gln Arg Pro Ser Arg Pro Glu Gln Gln Glu Pro Arg
325 330 335

Gly Arg Gly Cys Gln Thr Arg Asn Gly Val Glu Glu Asn Ile Cys Thr
340 345 350

Met Lys Leu His Glu Asn Ile Ala Arg Pro Ser Arg Ala Asp Phe Tyr
355 360 365

Asn Pro Lys Ala Gly Arg Ile Ser Thr Leu Asn Ser Leu Thr Leu Pro
370 375 380

Ala Leu Arg Gln Phe Gly Leu Ser Ala Gln Tyr Val Val Leu Tyr Arg
385 390 395 400

Asn Gly Ile Tyr Ser Pro Asp Trp Asn Leu Asn Ala Asn Ser Val Thr
405 410 415

Met Thr Arg Gly Lys Gly Arg Val Arg Val Val Asn Cys Gln Gly Asn
420 425 430

Ala Val Phe Asp Gly Glu Leu Arg Arg Gly Gln Leu Leu Val Val Pro
435 440 445

Gln Asn Pro Ala Val Ala Glu Gln Gly Gly Glu Gln Gly Leu Glu Tyr
450 455 460

Val Val Phe Lys Thr His His Asn Ala Val Ser Ser Tyr Ile Lys Asp
465 470 475 480

Val Phe Arg Val Ile Pro Ser Glu Val Leu Ser Asn Ser Tyr Asn Leu
485 490 495

Gly Gln Ser Gln Val Arg Gln Leu Lys Tyr Gln Gly Asn Ser Gly Pro
500 505 510

Leu Val Asn Pro
515

<210> 6
<211> 517
<212> PRT
<213> Glycinin (58.2 Kda)

<400> 6

Met Gly Lys Pro Phe Phe Thr Leu Ser Leu Ser Ser Leu Cys Leu Leu
1 5 10 15

Leu Leu Ser Ser Ala Cys Phe Ala Ile Thr Ser Ser Lys Phe Asn Glu
20 25 30

Cys Gln Leu Asn Asn Leu Asn Ala Leu Glu Pro Asp His Arg Val Glu
35 40 45

Ser Glu Gly Gly Leu Ile Glu Thr Trp Asn Ser Gln His Pro Glu Leu
50 55 60

Gln Cys Ala Gly Val Thr Val Ser Lys Arg Thr Leu Asn Arg Asn Gly
65 70 75 80

Leu His Leu Pro Ser Tyr Ser Pro Tyr Pro Gln Met Ile Ile Val Val
85 90 95

Gln Gly Lys Gly Ala Ile Gly Phe Ala Phe Pro Gly Cys Pro Glu Thr
100 105 110

Phe Glu Lys Pro Gln Gln Gln Ser Ser Arg Arg Gly Ser Arg Ser Gln
115 120 125

Gln Gln Leu Gln Asp Ser His Gln Lys Ile Arg His Phe Asn Glu Gly
130 135 140

Asp Val Leu Val Ile Pro Pro Gly Val Pro Tyr Trp Thr Tyr Asn Thr
145 150 155 160

```
Gly Asp Glu Pro Val Val Ala Ile Ser Leu Leu Asp Thr Ser Asn Phe
                165                 170                 175

Asn Asn Gln Leu Asp Gln Asn Pro Arg Val Phe Tyr Leu Ala Gly Asn
            180                 185                 190

Pro Asp Ile Glu His Pro Glu Thr Met Gln Gln Gln Gln Gln Gln Lys
        195                 200                 205

Ser His Gly Gly Arg Lys Gln Gly Gln His Gln Gln Gln Glu Glu Glu
    210                 215                 220

Gly Gly Ser Val Leu Ser Gly Phe Ser Lys His Phe Leu Ala Gln Ser
225                 230                 235                 240

Phe Asn Thr Asn Glu Asp Thr Ala Glu Lys Leu Arg Ser Pro Asp Asp
                245                 250                 255

Glu Arg Lys Gln Ile Val Thr Val Glu Gly Gly Leu Ser Val Ile Ser
            260                 265                 270

Pro Lys Trp Gln Glu Gln Glu Asp Glu Asp Glu Asp Glu Asp Glu Glu
        275                 280                 285

Tyr Glu Gln Thr Pro Ser Tyr Pro Pro Arg Arg Pro Ser His Gly Lys
    290                 295                 300

His Glu Asp Asp Glu Asp Glu Asp Glu Glu Glu Asp Gln Pro Arg Pro
305                 310                 315                 320

Asp His Pro Pro Gln Arg Pro Ser Arg Pro Glu Gln Gln Glu Pro Arg
                325                 330                 335

Gly Arg Gly Cys Gln Thr Arg Asn Gly Val Glu Glu Asn Ile Cys Thr
            340                 345                 350

Met Lys Leu His Glu Asn Ile Ala Arg Pro Ser Arg Ala Asp Phe Tyr
        355                 360                 365

Asn Pro Lys Ala Gly Arg Ile Ser Thr Leu Asn Ser Leu Thr Leu Pro
    370                 375                 380

Ala Leu Arg Gln Phe Gly Leu Ser Ala Gln Tyr Val Val Leu Tyr Arg
385                 390                 395                 400

Asn Gly Ile Tyr Ser Pro His Trp Asn Leu Asn Ala Asn Ser Val Ile
                405                 410                 415
```

Tyr Val Thr Arg Gly Lys Gly Arg Val Arg Val Val Asn Cys Gln Gly
420 425 430

Asn Ala Val Phe Asp Gly Glu Leu Arg Arg Gly Gln Leu Leu Val Val
435 440 445

Pro Gln Asn Phe Val Val Ala Glu Gln Gly Gly Glu Gln Gly Leu Glu
450 455 460

Tyr Val Val Phe Lys Thr His His Asn Ala Val Ser Ser Tyr Ile Lys
465 470 475 480

Asp Val Phe Arg Leu Ile Pro Ser Glu Val Leu Ser Asn Ser Tyr Asn
485 490 495

Leu Gly Gln Ser Gln Val Arg Gln Leu Lys Tyr Gln Gly Asn Ser Gly
500 505 510

Pro Leu Val Asn Pro
515

<210> 7
<211> 427
<212> PRT
<213> Basic 7S globulin (46.4Kda)

<400> 7

Met Ala Ser Ile Leu His Tyr Phe Leu Ala Leu Ser Leu Ser Cys Ser
1 5 10 15

Phe Leu Phe Phe Leu Ser Asp Ser Val Thr Pro Thr Lys Pro Ile Asn
20 25 30

Leu Val Val Leu Pro Val Gln Asn Asp Gly Ser Thr Gly Leu His Trp
35 40 45

Ala Asn Leu Gln Lys Arg Thr Pro Leu Met Gln Val Pro Val Leu Val
50 55 60

Asp Leu Asn Gly Asn His Leu Trp Val Asn Cys Glu Gln Gln Tyr Ser
65 70 75 80

Ser Lys Thr Tyr Gln Ala Pro Phe Cys His Ser Thr Gln Cys Ser Arg
85 90 95

Ala Asn Thr His Gln Cys Leu Ser Cys Pro Ala Ala Ser Arg Pro Gly
100 105 110

```
Cys His Lys Asn Thr Cys Gly Leu Met Ser Thr Asn Pro Ile Thr Gln
        115                 120                 125

Gln Thr Gly Leu Gly Glu Leu Gly Glu Asp Val Leu Ala Ile His Ala
    130                 135                 140

Thr Gln Gly Ser Thr Gln Gln Leu Gly Pro Leu Val Thr Val Pro Gln
145                 150                 155                 160

Phe Leu Phe Ser Cys Ala Pro Ser Phe Leu Val Gln Lys Gly Leu Pro
                165                 170                 175

Arg Asn Thr Gln Gly Val Ala Gly Leu Gly His Ala Pro Ile Ser Leu
            180                 185                 190

Pro Asn Gln Leu Ala Ser His Phe Gly Leu Gln Arg Gln Phe Thr Thr
        195                 200                 205

Cys Leu Ser Arg Tyr Pro Thr Ser Lys Gly Ala Ile Ile Phe Gly Asp
    210                 215                 220

Ala Pro Asn Asn Met Arg Gln Phe Gln Asn Gln Asp Ile Phe His Asp
225                 230                 235                 240

Leu Ala Phe Thr Pro Leu Thr Ile Thr Leu Gln Gly Glu Tyr Asn Val
                245                 250                 255

Arg Val Asn Ser Ile Arg Ile Asn Gln His Ser Val Phe Pro Leu Asn
            260                 265                 270

Lys Ile Ser Ser Thr Ile Val Gly Ser Thr Ser Gly Gly Thr Met Ile
        275                 280                 285

Ser Thr Ser Thr Pro His Met Val Leu Gln Gln Ser Val Tyr Gln Ala
    290                 295                 300

Phe Thr Gln Val Phe Ala Gln Gln Leu Pro Lys Gln Ala Gln Val Lys
305                 310                 315                 320

Ser Val Ala Pro Phe Gly Leu Cys Phe Asn Ser Asn Lys Ile Asn Ala
                325                 330                 335

Tyr Pro Ser Val Asp Leu Val Met Asp Lys Pro Asn Gly Pro Val Trp
            340                 345                 350

Arg Ile Ser Gly Glu Asp Leu Met Val Gln Ala Gln Pro Gly Val Thr
        355                 360                 365
```

```
Cys Leu Gly Val Met Asn Gly Gly Met Gln Pro Arg Ala Glu Ile Thr
    370                 375                 380

Leu Gly Ala Arg Gln Leu Glu Glu Asn Leu Val Val Phe Asp Leu Ala
385                 390                 395                 400

Arg Ser Arg Val Gly Phe Ser Thr Ser Ser Leu His Ser His Gly Val
                405                 410                 415

Lys Cys Ala Asp Leu Phe Asn Phe Ala Asn Ala
            420                 425
```